# EUROPEAN PATENT SPECIFICATION

(11) **EP 1 893 301 B1**
(45) Date of publication and mention of the grant of the patent: **21.03.2012**
(21) Application number: 06754396.7
(22) Date of filing: 16.06.2006
(51) Int. Cl.: A61Q 15/00, A61K 8/06, A61K 8/33, A61K 8/34, A61K 8/92, A61K 8/37

(54) **LOW-RESIDUE DEODORANT OR ANTIPERSPIRANT STICK BASED ON AN ETHANOL-CONTAINING OIL-IN-WATER DISPERSION/EMULSION**
NIEDRIGREST-DEODORANT ODER ANTIPERSPIRANS-STIFT AUF BASIS EINER ETHANOLHALTIGEN ÖL-IN-WASSER-DISPERSION/EMULSION
STICK DÉODORANT OU ANTITRANSPIRANT À FAIBLE RÉSIDU À BASE D UNE DISPERSION/ÉMULSION HUILE DANS L EAU CONTENANT DE L ÉTHANOL

(30) Priority: 24.06.2005 DE 102005029777; 30.03.2006 US 788023 P; 09.05.2006 DE 102006021780; 10.05.2006 US 799160 P
(43) Date of publication of application: 05.03.2008
(73) Proprietor: Henkel AG & Co. KGaA, 40589 Düsseldorf (DE)
(72) Inventor: BANOWSKI, Bernhard, 40597 Düsseldorf (DE); WADLE, Armin, 40699 Erkrath (DE); CLAAS, Marcus, 40723 Hilden (DE)
(86) International application number: PCT/EP2006/005787
(87) International publication number: WO 2006/136330

(56) References cited:
- DE-A1- 19 643 238
- DE-A1- 19 832 425
- US-A- 4 704 271
- US-A- 6 007 799
- US-A1- 2002 022 010
- US-A1- 2003 103 921

## Description

The invention relates to deodorant or antiperspirant sticks based on an ethanol-containing oil-in-water dispersion/emulsion for the application of active ingredients, in particular water-soluble active ingredients, to the skin.

Standard commercial deodorants and antiperspirants are mostly formulated as sprays or as sticks; there are also roll-on preparations and creams in the market. Many stick antiperspirant preparations are formulated as anhydrous suspension sticks. Preparations of this type leave behind a pleasant dry feel on the skin for the user following application. However, effective release of the water-soluble antiperspirant active ingredients from such preparations is limited (cf.: Chemistry and Technology of the Cosmetics and Toiletries Industry, edition D. F. Williams and W. H. Schmitt, London: Blackie, 1996, 2néd edition, p. 326), and in most cases the feeling of freshness valued by many consumers is not achieved. The anhydrous preparations, in particular those based on volatile silicone oils, have the disadvantage that the dispersed active ingredients readily lead to visible product residues on skin and clothing. Furthermore, such preparations are relatively expensive since the oil components are more expensive as active ingredient carriers than water. Compression during application often results in the loss of oil, which reduces the cosmetic acceptance of these preparations for the user.

Compared with anhydrous sticks, as are known, for example, from WO 94/24997 A1 and WO 00/67713 A1, emulsion sticks, as are disclosed, for example, in WO 98/17238 A1, EP 281 288 A2, DE 2 335 549, US 4,725,431, EP 617 952 A1 and EP 291 334, have a number of advantages. Replacing the wax and oil additives with water makes the emulsion sticks more cost-effective to manufacture. The emulsified waxes convey a soft, gentle feel on the skin, and, finally, water-soluble cosmetic active ingredients (i.e., in particular, antiperspirant active ingredients) can more readily be released onto the skin since they are already present in dissolved form in the aqueous phase of the emulsion.

WO 02/017870 A2 discloses antiperspirant sticks without a W/O emulsifier or high melting wax, which contain a siliconized polyamide as a consistency regulator or structurant. According to patent claim 1 of WO 02/017870 A2, the hydrous phase forms the internal phase, i.e. the dispersed phase, and the gels disclosed are water-in-oil emulsions.

WO 02/032914 A1 discloses, with reference to several exemplifying embodiments, hydrous antiperspirant sticks based on a water-in-oil emulsion, which contain acylated cellobiose as the consistency regulator or structurant and contain a high fraction of the silicone and hydrocarbon oils which are unfavorable according to the invention, and furthermore do not contain oil-in-water emulsifiers or a high melt wax.

Since the emulsion sticks of the cited prior art are formulated on the basis of a water-in-oil dispersion/emulsion, the water-soluble active ingredients are present in the inner, dispersed phase and, following application, must first migrate through the outer, lipophilic layer in order to reach their site of action on the skin. The known water-in-oil emulsion sticks thus have disadvantages, which are similar to those of anhydrous suspension sticks with regard to the availability of active ingredient.

DE 19749819 A1 discloses water-containing and oil-containing, wax-free antiperspirant sticks based on an oil-in-water emulsion. Sticks of this type have inadequate cosmetic properties, leave behind unpleasant sticky and visible residues and exhibit a stability, which is insufficient for prolonged use. One example with glycerol monostearate as W/O emulsifier and octyldodecanol as oil component has a medium-firm consistency and a greasy feel on the skin and begins to soften at just 50° C.

WO 99/59537 A1 discloses hydrous cosmetic sticks which comprise wax components with a melting point of >50° C, nonionic water-in-oil emulsifiers, a nonionic oil-in-water emulsifier with an HLB value of more than 7 and a polyol. Some of the sticks contain oils which are liquid at 25° C but which, instead of being incorporated at the beginning of the emulsion process as in the sticks of the present application, are stirred in as a pre-emulsified concentrate, for instance a micro-emulsion or PIT emulsion, during the cooling phase of the stick mass at a temperature of 55° C. This type of production method is needed in order not to endanger or even destroy the stability of the system for a dispersion of lipid and wax crystals. Sticks of this type likewise have inadequate cosmetic properties, can leave behind unpleasant sticky and visible residues, and exhibit a stability, which is inadequate for prolonged use.

WO 02/083091 A1 discloses structured antiperspirant compositions in the form of a microemulsion which represents an oil-in-water microemulsion or a water-in-oil microemulsion or a bicontinuous phase, depending on the kind and quantity of surfactants, but in which the bicontinuous phase predominates overall. The (transparent) microemulsions are condensed by an oil-soluble or oil-dispersible "structurant." The oil-soluble or oil-dispersible "structurant" is chosen from among esters and amides of 12-hydroxystearic acid, esters and amides of di- and tricarboxylic acids, sterols, sterol esters such as oryzanol, cellobiose fatty acid esters, sugar esters such as acylated maltose, and non-crosslinked oil-soluble or oil-dispersible polymer oil phase condensing agents such as the commercial product Kraton G. Nonionic emulsifiers with an HLB value from 2-15, preferably with an HLB value under 12, are also incorporated. Polyols are disclosed as optional only. This document does not disclose the possible significance of the solubility parameters of W/O emulsifiers and oil components being matched with one another. The structural difference between these compositions and the oil-in-water dispersion/emulsion sticks of the present invention, which are not microemulsions, becomes particularly clear because of the high fraction, namely 19-66% by weight relative to the overall composition, of silicone and (paraffinic) hydrocarbon oils which all exemplifying embodiments disclose but which are unfavorable according to the present invention.

The published applications DE 199 62 878 A1 and DE 199 62 881 A1 disclose deodorant or antiperspirant creams based on an oil-in-water emulsion which have, at 21° C, a viscosity of at least 50,000 mPas, preferably in the range from 200,000-1,500,000 mPas, i.e., they are in viscous to highly viscous paste form. These creams comprise wax components with a melting point of >50° C, nonionic water-in-oil emulsifiers, nonionic oil-in-water emulsifiers with an HLB value of more than 7, and a polyol. Being soft creams, they can be applied either by using only the fingers, which is rejected by many consumers as being impractical, or by pouring the creams into special applicators, which are significantly more expensive than the stick sheaths for the deodorant or antiperspirant sticks according to the invention. If, after being heated and mixed, the compositions disclosed in DE 199 62 878 A1 and DE 199 62 881 A1 were cooled statically, i.e. without stirring, then stick-like compositions would be obtained which have overall unfavorable application properties, such as poor haptics and/or inadequate stability, for example as a result of phase separation or the formation of water condensation, since the emulsifiers and the oils are not matched to one another as in the present invention.

The not prior-published application DE 10 2004 036 689.6 disclose deodorant or antiperspirant sticks in the form of an oil-in-water dispersion containing at least one lipid or wax component with a melting point of > 50° C, at least one non-ionic oil-in-water emulsifier with an HLB value above 7 within a nonionic oil-in-water emulsifier system with an average HLB value between 10 and 19; as a consistency regulator and/or water binder, at least one nonionic water-in-oil emulsifier with an HLB value of greater then 1.0 and less than/equal to 7.0, which can form liquid crystalline structures with water alone or with water in the presence of a hydrophilic emulsifier, [and] at least one oil which is in a liquid state at 20° C and is not a fragrance component or essential oil -- the maximum deviation between the (average) solubility parameter of all the constituent oils and the (average) solubility of the water-in-oil emulsifier or emulsifiers being -0.7 (cal/cm³)^{0.5} or +0.7 (cal/cm³)^{0.5} in the presence of linear saturated fatty alcohol as the water-in-oil emulsifier or part of a water-in-oil emulsifier, respectively, and -0.4 (cal/cm³)^{0.5} or +0.7 (cal/cm³)^{0.5} in the presence of water-in-oil emulsifiers other than linear saturated fatty alcohols in the absence of linear saturated fatty alcohols as a water-in-oil emulsifier, respectively; at least one water-soluble polyhydric C₂ - C₉ alkanol with 2 - 6 hydroxyl groups and/or at least one water-soluble polyethylene glycol with 3-20 ethylene oxide units; 5% to less than 50% by weight of water relative to the whole composition; and at least one deodorant or antiperspirant agent; where the stick exhibits a penetration force value in the range of 200-600 gram-force (g-force) at a depth of 5.000 mm (five millimetres) and a maximum electrical resistance of 300 kΩ (Kiloohm).

The object was to develop a deodorant or antiperspirant composition, which is suitable as an effective carrier for active ingredients, in particular water-soluble active ingredients, and permits the rapid release of the active ingredient on the skin.

A further object was to develop a deodorant or antiperspirant composition with excellent cosmetic care properties.

A further object was to develop a deodorant or antiperspirant stick which, on the one hand, has high stability, i.e., solidity, but on the other hand, has a pleasant release behavior respectively rub-off behavior, i.e. is not too solid but can be readily spread over the skin and in so doing releases an adequate amount of product.

A further object was to develop a deodorant or antiperspirant composition which, when applied to the skin, leaves behind as little sticky or visible residue as possible.

A further object was to develop a deodorant or antiperspirant composition, which leaves behind optimally little visible residue on clothing which comes into contact with the treated skin.

A further object was to develop a deodorant or antiperspirant composition, which can be readily washed off of the skin.

A further object was to develop a deodorant or antiperspirant composition with a cost-performance ratio, which is favorable economically and in terms of application.

A further object was to develop a deodorant or antiperspirant composition, which allowed for the mass production of stable deodorant or antiperspirant sticks with a suitable consistency.

Surprisingly and unforeseeably to the person skilled in the art, these objects were achieved through a deodorant or antiperspirant stick in the form of an oil-in-water dispersion/emulsion comprising

a) at least one lipid or wax component with a melting point of >50° C which is not to be apportioned to the components b) or c),

b) at least one nonionic oil-in-water emulsifier with an HLB value of more than 7 within an oil-in-water emulsifier mixture with an average HLB value in the range of 10-19,

c) at least one nonionic water-in-oil emulsifier with an HLB value greater than 1.0 and less than or equal to 7.0, which can form liquid crystalline structures with water alone or with water in the presence of a hydrophilic emulsifier, as a consistency regulator and/or water binder;

d) at least one oil which is in a liquid state at 20° C and is not a fragrance component or essential oil, in which the maximum deviation between the (average) solubility parameter of all the constituent oils d) and the (average) solubility parameter of the water-in-oil emulsifier or emulsifiers is -0.7 (cal/cm³)^{0.5} or +0.7 (cal/cm³)^{0.5}, respectively, in the presence of linear saturated fatty alcohols with a chain length of at least 8 carbon atoms and -0.4 (cal/cm³)^{0.5} or +0.7 (cal/cm³)^{0.5}, respectively, in the presence of water-in-oil emulsifiers other than linear saturated fatty alcohols with a chain length of at least 8 carbon atoms, linear saturated fatty alcohols with a chain length of at least 8 carbon atoms being absent and the share of silicon oils and/or hydrocarbons being not more than 20% in relation to the total weight of oils, which are liquid at 20°C,

e) at least one water-soluble polyhydric C₂-C₉-alkanol with 2-6 hydroxyl groups and/or at least one water-soluble polyethylene glycol with 3-20 ethylene oxide units,

f) 5% to less than 50% by weight of water, relative to the overall composition,

g) 1% to 15% by weight of ethanol and/or isopropanol, relative to the overall composition,

h) at least one deodorant or antiperspirant active ingredient.

The lipid or wax component with a melting point of >50° C forms a gel matrix with the oil(s) and optionally further higher-melting lipid or wax components; this gel matrix can absorb larger amounts of water, ethanol and/or isopropanol and polyol. These structures, which are stabilized by certain amounts of water-in-oil emulsifiers and oil-in-water emulsifiers, leave behind a fresh, cooling impression upon application due to their water content and, in particular, due to their ethanol and/or isopropanol content. Here, the emulsifiers are matched to one another so that the stick compositions according to the invention are present in the form of an oil-in-water dispersion/emulsion. The stick compositions of the invention are thus not present as a microemulsion. To produce the stick compositions of the invention, the water phase and the oil phase must be heated to at least 70° C and stirred together or homogenized while hot, i.e. at least at 70° C, in order to achieve the emulsion structure of the invention. A production method like the one disclosed in US 4,205,062, for example (kneading of fat and water phase at 65° C) is inadequate to obtain a homogenous stick composition based on an oil-in-water dispersion/emulsion. Without wishing to be bound to this theory, it is assumed that the oil-in-water emulsifiers, together with some of the water-in-oil emulsifiers, form lamellar liquid crystal phases, which are built up with some of the water into a hydrophilic gel phase. This hydrophilic gel phase surrounds the aqueous bulk phase. Dispersed within this aqueous bulk phase are, in turn, the lipophilic components, surrounded by a lipophilic gel phase, which is formed by the water-in-oil emulsifiers with some of the oil-in-water emulsifiers and some water.

The antiperspirant active ingredient is dissolved in the outer, continuous aqueous phase, resulting in a considerably improved and more efficient active ingredient release compared to the known anhydrous suspension sticks and water-in-oil emulsion sticks. The O/W emulsion basis of the stick compositions of the invention results in a considerably improved and more efficient active ingredient release compared to the known anhydrous suspension sticks and water-in-oil emulsion sticks. This active ingredient release can be determined indirectly very readily by measuring the electrical resistance of the particular product. Measuring the electrical resistance of such compositions is also a suitable way to be able to distinguish between an oil-in-water system and a water-in-oil system. An oil-in-water system exhibits a high electrical conductivity and therefore a low electrical resistance owing to the continuous water phase. The precise measurement set-up and the measurement procedure are described below (see below). The sticks according to the invention accordingly have an electrical resistance preferably of at most 300 kΩ, more preferably of at most 100 kΩ, and particularly preferably of at most 80 kΩ. In contrast, the sticks disclosed in WO 98/17238 A1 exhibit an electrical resistance of more than 3,000 kΩ; therefore, they obviously employ a water-in-oil system.

The solidification of the deodorant or antiperspirant sticks according to the invention does not take place on the basis of soap gels or fatty acid salt gels, fatty acids being understood as meaning alkanoic, alkenoic and alkinoic acids having at least 4 carbon atoms, which can be substituted, for example, by hydroxyl groups. In a particularly preferred embodiment, the deodorant or antiperspirant sticks according to the invention are free of soap gels or fatty acid salt gels, in particular, free of lithium, sodium, potassium, ammonium, diethanolamine and triethanolamine salts of fatty acids. Sticks on a soap base are incompatible with acidic antiperspirant active ingredients such as are used in the antiperspirant sticks of the invention.

The solidification of the deodorant or antiperspirant sticks according to the invention does not take place on the basis of inorganic and/or organic polymeric hydrogel formers, such as celluloses, cellulose derivatives, for example hydroxyalkylcelluloses, polyacrylates, veegum or bentones. In a particularly preferred embodiment, the deodorant or antiperspirant sticks according to the invention are free of gels formed by inorganic and/or organic polymeric hydrogel formers.

Besides the favorable active ingredient release, the formulation as ethanol-containing oil-in-water dispersion/emulsion is accompanied by further advantages. First, the composition can be readily washed off of the skin. Second, during or following application to the skin, a caring oil-in-water cream forms together with the skin moisture causing a refreshing, cooling skin sensation.

Surprisingly and unexpectedly to the person skilled in the art, it has been found that the oil components and the water-in-oil emulsifier or the water-in-oil emulsifier mixture have to be matched to one another with regard to their solubility parameters in order to form stick compositions with satisfactory performance-related hardnesses. For a definition of the solubility parameter within the meaning of the present invention, reference is made to the publication "Solubility-Effects in Product, Package, Penetration and Preservation," by Chr. D. Vaughan in Cosmetics & Toiletries, vol. 103, October 1988, pages 47-69. The values for the solubility parameters published therein are noted in the non-SI unit (cal/cm³)^{0.5}. For the sake of simplicity, this non-SI unit will be retained in this specification. The values can be easily converted based on the relation 1 cal = 4,1860 Joules.

A number of the solubility parameters tabulated by Vaughan in Cosmetics & Toiletries, Vol. 103, October 1988, pages 47 - 69, were calculated according to the Hildebrand equation (see C. D. Vaughan: J. Soc. Cosmet. Chem., Vol. 36, pp. 319 - 333 (Sept./Oct. 1985) and the Hildebrand equation cited therein, and J. Am. Chem. Soc., Vol. 38, pages 1442 - 1473 (1916) and J. Hildebrand and R. Scott: The Solubility of Nonelectrolytes, 3rd Edition, Reinhold Publ. Corp., New York, 1949); they are summarized below. Vaughan mentions that the solubility parameters can be calculated not only using the Hildebrand equation but also, for example, based on the evaporation enthalpy (Scatchard, J. Am. Chem. Soc., Vol. 38, page 321 (1916)). All the calculation methods can produce different values for the solubility parameters, especially if the chemical material has an acid or base function.

In the present invention, it is preferable when the matching of the solubility parameters of the oil components and the water-in-oil emulsifier or the water-in-oil emulsifier mixture is performed only for solubility parameter values that were calculated using the same method. It is particularly preferred when the solubility parameter values that were calculated using the Hildebrand equation ((see C.D. Vaughan: J. Soc. Cosmet. Chem., Vol. 36, pages 319 - 333 (Sept./Oct. 1985)) are used for the matching according to the invention. If there is no available pair of solubility parameter values that were determined using the same method for a particular combination of oil component and water-in-oil emulsifier, it is also possible to use values that were determined using different methods, even experimental ones. However, that is a less preferred alternative according to the invention.

**Table 1: Solubility parameter of various chemical components (from Cosmetics & Toiletries, Vol. 103, October 1988, pages 47 - 69)**

| MATERIAL NAME (CTFA) | Solubility | Ref. | MATERIAL NAME (CTFA) | Solubility Parameter (cal/c m³)^{0.5} | Ref. |
|---|---|---|---|---|---|
| with Dielectric Constant | Parameter (cal/cm³)^{0.5} | | with Dielectric Constant | | |
| | | | Cyclohexane (2.02) | 7.30 | E |
| | | | Dioctyl Ether | 7.30 | A |
| | | | Eicosane (020) | 7.32 | C |
| Helium (1.06) | 0.50 | *N | Lanolin Oil | 7.33 | L1 |
| Hydrogen (1.23) | 2.50 | *N | Petrolatum | 7.33 | *0 |
| Propellant 13 | 2.59 | *0 | Behenic Acid | 7.35 | 10 |
| Methane (1.70) | 4.70 | *0 | Diethyl Ether (4.34) | 7.37 | CO |
| Neon | 4.90 | *N | Corn Oil-Refined | 7.40 | L1 |
| Perfluorohexane | 5.68 | A | Cetane (016) | 7.41 | I |
| Perfluoroctane | 5.72 | A | Heptane (1.92) | 7.41 | CO |
| Cyclomethicone D5 (2.50) | 5.77 | MO | Isostearyl Neopentanoate | 7.43 | M |
| Nitrogen (1.45) | 5.90 | *N | Octyl Palmitate | 7.44. | 0 |
| Dimethicone | 5.92 | *0 | Propyl Fluoride | 7.48 | C |
| Cyclomethicone D4 (2.39) | 5.99 | MO | Rice Oil - SO | 7.48 | L1 |
| Squalane | 6.03 | MO | Tridecane (C13) | 7.48 | CO |
| Propellant 12 (2.13) | 6.11 | *0 | Propellant 11 (2.28) | 7.49 | 0 |
| Hexamethyldisiloxane (2.17) | 6.15 | MO | Cottonseed Oil | 7.52 | L1 |
| Isocetyl Stearate | 6.19 | M | Carbon Dioxide (1.60) | 7.53 | H |
| Squalene | 6.19 | MO | Isopropyl Linoleate | 7.55 | M |
| Polytetrafluoroethylene | 6.20 | * | Cod Liver Oil | 7.56 | L1 |
| Propane | 5.21 | *0 | Erucic Acid | 7.57 | CO |
| Propellant 22(6.11) | 6.23 | MO | Octane (1.95) | 7.58 | MO |
| Perfluorodecalin | 6.34 | A | Cetyl Octanoate | 7.59 | M |
| Neopentane | 6.38 | CO | Decene-1 | 7.59 | C |
| Safflower Oil | 6.42 | L1 | Dodecene (2,01) (7.65-I) | 7.59 | C |
| Melene (C30) | 6.58 C | C | Diethylhexyl Adipate | 7.60 | M |
| Docosane (C22) | 6.60 | I | Decane (1.99) | 7.62 | CO |
| Almond Oil | 6.81 | L1 | C12-15 Alcohols Benzoate | 7.63 | MO |
| Isopentane | 6.82 | CO | Isobutyl Stearate | 7.65 | 0 |
| Avocado Oil | 6.83 | L1 | Butyl Myristate | 7.68 | D |
| Nonacosane (D29) | 6.83 | C | Butyl Stearate(3.11) | 7.68 | CO |
| Arachidic Acid | 6.85 | H | Stearic Acid (C18)(2,30) | 7.74 | IO |
| Pristane | 6.85 | MO | Dioctyl Maleate | 7.75 | 0 |
| Decyl Oleate | 6.92 | M | Octyl Fluoride | 7.76 | AG |
| C8-Isoparaffin (1.94) | 6.93 | MO | Isopropyl Palmitate | 7.78 | 0 |
| Diisopropyl Ether (3.88) | 6.95 | KE | Dioctyl Adipate | 7.82 | M |
| Argon (1.53) | 7.00 | *N | Oleth-3 | 7.83 | *0 |
| Sperm Oil | 7.09 | *0 | Diethyl Amine | 7.86 | C |
| White Mineral Oil | 7.09 | *0 | Linolenic Acid | 7.86 | C0 |
| Pentane | 7.10 | *0 | Olive Oil | 7.87 | *0 |
| Tricosane (C23) | 7.13 | C | Palmitic Acid (C16) (22.30) | 7.89 | IO |
| Isodecyl Oleate | 7.17 | M | Oleic Acid(2,46) | 7.91 | IO |
| Propellant 113 | 7.19 | H | PEG-4 Stearate | 7.92 | 0 |
| Oxygen (1.50) | 7.20 | *N | Tetraethyl Lead | 7.92 | E |
| Cholesteryl Oleate | 7.24 | * | Tridecyl Neopentanoate | 7.92 | L1 |
| Peanut Oil | 7.74 | L1 | Pentaerythrityl Tetraoleate | 7.98 | L1 |
| Hexane (1.88) | 7.28 | CO | Tocopheryl Acetate | 7.98 | M |
| Linseed Oil | 7.29 | *0 | Ethyl Myristate | 8.00 | C |
| Octadecane (C18) | 7.29 | C | | | |
| Isopropyl Myristate | 8.02 | 0 | Stearyl Alcohol (C18) | 8.90 | I0 |
| Turpentine (pinene)(2.70) | 8.03 | CO | Methyl Hexyl Ketone | 8.91 | A |
| Human Erythrocyte | 8.05 | * | Octyl Dodecanol | 8.92 | OM |
| Methyl Oleate (3.21) | 8.05 | CO | Butyl Acetate (5.01) | 8.93 | CO |
| Cetyl Acetate | 8.06 | 0 | Cetyl Alcohol (CIG) | 8.94 | I0 |
| Methyl Linoleate | 8.08 | C | alpha-Thujone | 8.94 | A |
| Isostearic Acid | 8.09 | 0 | Toluene (2.38) | 8.94 | C |
| Coconut Oil | 8.10 | L1 | Oleyl Alcohol | 8.95 | CO |
| Myristic Acid (C14) | 8.10 | I0 | Propylene Oxide | 8.99 | A |
| Dibutylamine | 8.15 | * | Aspergillus Niger | 9.00 | P |
| Eucalyptol (Cineole) | 8.17 | L1 | Octyl Dimethyl PABA 9.34 G | 9.01 | OM |
| Natural Rubber | 8.20 | H | Propyl Acetate | 9.02 | CO |
| Octylamine | 8.21 | A | Chloroform | 9.05 | A |
| Propylene Glycol Dipelargonate | 8.21 | L1 | Benzene (2,28) | 9.08 | E |
| Titanium Isopropoxide | 8.21 | M | PEG-20 Stearate | 3.08 | J3 |
| Melissyl Alcohol (C 30) | 8.22 | CO | Ceteth-20 | 9.10 | H |
| Glycol Distearate | 8.24 | J3 | Methyl Butyl Methacrylate CO | 8.10 | M |
| Glycol Stearate | 8.28 | J3 | Octyl Methoxycinnamate | 9.10 | M |
| Capric/Caprylic Triglycerid | 8.29 | L1 | Methyl Butyl Ketone | 9.11 | E |
| Isosteareth-2 | 8.29 | L1 | Myristyl Alcohol (C14) | 9.16 | IO |
| PPG-2 Myristyl Ether | 8.29 | L1 | Polysorbate-20 | 9.16 | J3 |
| Ricinoleic Acid | 8.30 | C | THF (7.58) | 9.16 | E |
| Staphylococcus Aureus | 8.30 | P | BHT | 9.17 | D |
| Glyceryl Isostearate | 8.31 | J3 | Tocopherol | 9.17 | M |
| Glyceryl Stearate (mono) | 8.31 | *0 | Lauryl Lactate | 9.18 | M |
| Laureth-4 | 3.31 | J3 | PEG-40 Stearate | 9.18 | J3 |
| Limonene (2,30) | 8.33 | C | Ethyl Acetate (6.02) | 9.19 | CO |
| Propylene Glycol Laurate | 8.33 | L1 | Tributyl Citrate | 9.20 | M |
| Octyl Mercaptan | 3.35 | K | Ethyl Acrylate | 9.22 | A |
| PEG-2 Stearate | 8.36 | J3 | Propionaldehyde | 9.22 | A |
| Ethyl Caprate (C10) | 8.39 | A | Methyl Propyl Ketone | 9.27 | C |
| Radon | 8.40 | *N | Dipropyl Nitrosamine | 9.29 | B |
| Amyl Acetate | 8.43 | C | alpha-Bisabolol | 9.30 | M |
| Glyceryl Stearate SE | 8.43 | J3 | Pseudomonas Aeroginosa | 9.30 | P |
| Diisopropyl Adipate | 8.46 | E0 | Trichomonas Ment. | 9.30 | P |
| Lauric Acid (C12) | 8.46 | I0 | Caprylic Acid (C8)(2.45) | 9.32 | E0 |
| Polyethylene (2,35) | 8.50 | *0 | Cetyl Lactate | 9.32 | M |
| Diisopropyl Amine | 8.51 | *0 | PEG-100 Stearate | 9.35 | J3 |
| Polyglyceryl-3 Oleate | 8.52 | J3 | Trimethyl Citrate | 9.39 | H |
| EthyleneNinyl Acetate (AC400) | 8.55 | *0 | Klebsiella Pneumoniae | 9.40 | P |
| Ethyl Caprylate (C8) | 8.57 | A | Methyl Methacrylate Copolymer | 9.40 | H |
| Octyl Acetate | 8.58 | A | Nicotine | 9.40 | c |
| Octyl Iodide | 8.58 | A | Camphor | 9.45 | C |
| Ethyl Oleate (3.17) | 8.60 | * | Oxidized Polyethylene (AC392) | 9.50 | *0 |
| Isopropylbenzene (12.38) | 8.60 | * | Lauryl Alcohol (C12) | 9.51 | C0 |
| Sorbitan Laurate | 8.61 | 0 | Pulegone | 9.51 | A |
| Behenyl Alcohol (C22) | 8.63 | I0 | Cholesterol | 9.55 | 0 |
| Carbon Tetrachloride (2.23) | 8.64 | C | Ethylene/Vinyl Acetate (AC430) | 9.55 | *0 |
| Butyl Mercaptan | 8.65 | KA | Methylene Chloride (9.08) | 9.55 | E |
| Isostearyl Alcohol | 8.67 | 0 | Dimethyl Isosorbide | 9.58 | M |
| Lauraldehyde | 8.68 | A | PPG-2 Methyl Ether | 9.60 | * |
| Ethyl Caproate (C6) | 8.69 | A | Acetaldehyde (21.8) | 9.61 | A |
| Cholesteryl Propionate | 8.70 | * | Undecyl Alcohol | 9.51 | C0 |
| Isocetyl Alcohol | 8.71 | M | Linalool | 9.62 | C |
| Bornyl Acetate | 8.74 | CA | Methyl Ethyl Ketone (18.50) 9.53A | 9.63 | C0 |
| Ethyl Mercaptan | 8.75 | K | Acetylacetone | 9.68 | * |
| Decanone-2 | 8.76 | A | Amyl Dimethyl PABA | 9.72 | M |
| Octanal | 8.77 | C | Methyl Iodide | 9.75 | C |
| Trifluoroactylacetone | 8.77 | A | Decyl Alcohol (C10) (8.10) | 9.78 | C0 |
| Cholesteryl Myristate | 8.80 | * | Chlorine | 9.80 | *H |
| Zinc Stearate | 8.80 | 0 | Ethylhexanol | 9.80 | A |
| Citronella | 8.83 | C0 | Stratum Corneum-Porcine | 9.80 | * |
| Diethyl Ketone (17.00) | 8.85 | E | Acetone (20.70) | 9.87 | C |
| Methyl Isobutyl Ketone (14.70) | 8.85 | E0 | Citronellol | 9.88 | A |
| Oxidized Polyethylene (AC629) | 8.85 | *0 | Dibutyl Phthalate (6.44) | 9.88 | M |
| Methyl Heptyl Ketone | 8.86 | A | Menthyl Anthranilate | 9.89 | M |
| Myristyl Lactate | 8.87 | M | PPG-4 | 9.89 | M |
| Capric Acid (C10) | 8.88 | I0 | Ethoxyethanol (29.60) | 9.90 | *M |
| Methyl Caproate (CB) | 8.88 | B | Ethylene Oxide (13.90) | 9.93 | A |
| Arachidyl Alcohol (C20) | 8.89 | CO | Menthol | 9.94 | C0 |
| Dipropyl Ketone | 8.89 | C | Tributyrin | 9.97 | 0 |
| Muscone 8.89 | 8.89 | CO | Butoxydiglycol-BuCarbitol | 9.98 | * |
| Candida Albicans | 8.90 | P | Nitrous Oxide (1.60) | 10.00 | *H |
| Castor Oil | 8.90 | H | Dioxane (2.21) | 10.01 | * |
| Elaidyl Alcohol | 8.90 | CO | Ethyl Benzoate (6.02) | 10.01 | C |
| beta-Ionone | 8.90 | CO | Caproic Acid (C8) (2.53) | 10.05 | E0 |
| Polystyrene | 8.90 | M | Salicylic Acid | 10.06 | C |
| Nicoteine | 10.08 | C | Copper Acetylacetonide | 11.60 | * |
| Octanol/Caprylic (C8) Alcohol (10.34) | 10.09 | CO | | | |
| Acetic Anhydride (22.40) | 10.12 | C | Sulfamethoxazole | 11.60 | J1 |
| Nerol | 10.13 | C | PEG-4 (20.44) | 11.61 | DO |
| Ethyl Cinnamate | 10.14 | A | Acetohexamide | 11.64 | * |
| Diethyl Nitrosamine | 10.16 | C | N-Methylpyrrolidone | 11.71 | A |
| Octyl Salicylate | 10.17 | M | Propyl Alcohol (20.10) | 11.73 | CO |
| Griseofulvin | 10.20 | M | Dimethyl Nitrosamine | 11.74 | C |
| Dioctyl Malate | 10.21 | M | Pentobarbital | 11.75 | J1 |
| Geraniol | 10.21 | CO | Butadiene Diepoxide | 11.78 | A |
| Butyl Lactate | 10.27 | AO | Dipropylene Glycol (PPG-2) | 11.78 | M |
| t-Butyl Alcohol (10.90) | 10.28 | CO | Phthalide | 11.78 | C |
| Morpholine (7.33) | 10.28 | C | Lysine | 11.79 | J1 |
| Homosalate | 10.29 | GM | Phenethyl Alcohol | 11.79 | CO |
| Valeric Acid (C5) | 10.29 | A | Acetonitrile (37.5) | 11.81 | AO |
| Polyethylene Terephthalate (PET) | 10.30 | * | Cinnamic Acid | 11.83 | C |
| Pyridine (12.3) | 10.30 | A | p-Nitrotoluene (24.20) | 11.83 | |
| Phenyl Acetate (5.23) | 10.33 | E | Phenoxyethanol | 11.87 | CO |
| Thiolacetic Acid | 10.38 | A | Butobarbital | 11.90 | J1 |
| Methoxypropanol | 10.40 | * | Sulfadiazine | 11.90 | * |
| Diethyl Toluamide | 10.46 | M | Butalbital | 11.95 | J1 |
| Nonoxynol-1 | 10.47 | * | Cinnamyl Alcohol | 11.96 | C |
| Borneol | 10.48 | C | Sorbic Acid | 11.97 | MO |
| Methyl Benzoate (6.59) | 10.48 | E | Methylparaben | 11.98 | 0 |
| Hexyl Alcohol (13.30) | 10.50 | 10 | Hydroxyanisole | 12.00 | C |
| SAN (85/15) | 10.50 | * | Benzocaine | 12.05 | * |
| Butoxyethanol (9.30) | 10.53 | E | Triethylene Glycol (23.69) | 12.21 | MO |
| Formaldehyde | 10.54 | C | Alanine | 12.23 | J1 |
| o-Nitrotoluene (27.40) | 10.55 | B | Nitromethane | 12.27 | C |
| Butylparaben | 10.57 | * | Benzyl Alcohol (13.10) | 12.31 | 0 |
| Propionitrile | 10.57 | A | Hexylene Glycol | 12.32 | * |
| Tripropylene Glycol (PPG-3) | 10.60 | M | Butyramide | 12.33 | A |
| Methyl Salicylate (9.41) | 10.62 | C0 | Human Serum Albumin A | 12.33 | J1 |
| Acetophenone (17.39) | 10.64 | C | Vanillin | 12.34 | D |
| Diacetone Alcohol (18.20) | 10.67 | CO | BHA | 12.37 | 0 |
| Ethyl Anthranilate | 10.67 | C | Acetic Acid (6.15) | 12.40 | CO |
| Naphthylene | 10.74 | B | Cyclobarbital | 12.40 | J1 |
| Phenylpentanol | 10.74 | A | Diisopropanolamine | 12.40 | A |
| Butyric Acid (2.97) | 10.75 | E | Ethyl Dihydroxypropyl PABA | 12.42 | M |
| Cyclopentanone | 10.77 | E | o-Propylene Diamine | 12.43 | D |
| Thymol | 10.77 | C | p-Dinitrobenzene | 12.49 | B |
| Triacetin | 10.77 | 0 | Ethyl Alcohol (24.30) | 12.55 | CO |
| Methoxyethanol (16.90) | 10.80 | * | Rat Gut Membrane | 12.60 | * |
| Amyl Alcohol (13.90) | 10.84 | CE | Sulfamethazine | 12.60 | J1 |
| Ethanedithiol | 10.87 | A | Sulfisomidinc | 12.70 | J1 |
| Ethyl Hexanediol | 10.89 | A | Sulfur (3.55) | 12.70 | *N |
| Trichloroacetic Acid | 10.89 | E | Phenol (9.78) | 12.79 | CE |
| Benzalphthalide | 10.90 | 0* | Sulfisomidine | 12.80 | * |
| Testosterone | 10.90 | * | Allobarbital | 12.85 | J1 |
| Cinnamaldehyde | 10.92 | C | o-Nitroaniline (34.50) | 12.88 | D |
| Propylparaben | 10.94 | GM | Pyruvic Acid | 12.94 | * |
| Valine | 10.94 | J1 | Phenobarbital | 13.00 | J1 |
| Tolbutamide | 10.98 | * | Isopropanolamine | 13.02 | A |
| Benzaldehyde (17.80) | 11.00 | CO | Adipic Acid | 13.04 | 0 |
| Triisopropanolamine | 11.02 | M | BAL (2,3-Dimercapto-1-propanol) | 13.10 | B |
| Phenylbutanol | 11.04 | A | Sulfathiazole | 13.10 | * |
| Eugenol | 11.12 | C | Aminoethyl Ethanolamine | 13.18 | M |
| D&C Red 22(Eosin) | 11.15 | L2 | Glutathione | 13.18 | G |
| Butyl Alcohol (17.51) | 11.18 | C0 | Butylene Glycol | 13.20 | CO |
| Cellulose Acetate | 11.20 | H | m-Nitroaniline | 13.23 | C |
| Methyl Anthranilate | 11.22 | C | Triethanolamine (29.36) | 13.28 | MO |
| Caproamide (C6) | 11.24 | M | Propylene Carbonate (65,00) | 13.35 | * |
| Isopropyl Alcohol (18,30) | 11.24 | C0 | Benzamide | 13.38 | B |
| Nitrocellulose | 11.25 | M0 | Dimethyl Sulfoxide (46.68) | 13.40 | H |
| Hexobarbital | 11.30 | J1 | Sulfamerazine | 13.40 | 31 |
| Secobarbital | 11.30 | J1 | Propionamide | 13.46 | AC |
| p-Anisaldehyde | 11.32 | A | Barbital | 13.50 | J1 |
| PEG-8 | 11.34 | MO | Mercaptoethanol | 13.55 | A |
| Panthenol | 11.39 | MO | Propiolactone | 13.56 | A |
| Propionic Acid (3.35) | 11.40 | EA | Diethylene Glycol (31.70) | 13.61 | E0 |
| Glyoxal | 11.46 | C | Propargyl Alcohol | 13.61 | A |
| Phenylpropanol | 11.46 | A | p-Nitroaniline (56,30) | 13.67 | A |
| Methyl Lactate | 11.47 | CO | Caffeine | 13.80 | * |
| PEG-6 (16.00) | 11.47 | D0 | Thiodiglycol | 13.80 | M |
| Benzoic Acid (Chameleonic) | 11.50 | * | Thioglycolic Acid | 13.86 | A |
| PEG-5 (18.16) | 11.54 | D0 | Sulfameter | 13.90 | J1 |
| Phenylalanine | 11.57 | G | Diethanolamine | 13.95 | M |
| Propylene Glycol (32.00) | 14.00 | CO | Pyrrolidone | 14.00 | * |
| Theophyllin | 14.00 | * | Hexyl Resorcinol | 14.06 | * |
| Aspartic Acid | 14.11 | J1 | Sodium Lauryl Sulfate | 14.18 | * |
| Pyrrolidinone-2 | 14.22 | | Methyl Alcohol (32.70) | 14.33 | CO |
| Ethylene Glycol(37.00) | 14.50 | CO | Urea | 14.50 | G |
| Hydroquinone | 14.62 | | Formic Acid (58.5) | 14.72 | E |
| Lactic Acid (22.00) | 14.81 | | PABA 14.56G | 14.82 | DO |
| Resorcinol | 14.96 | C | Acetamide MEA | 15.11 | M |
| Histidine | 15.25 | J1 | p-Hydroxybenzoic Acid | 15.30 | * |
| Ethanolamine (37.72) | 15.41 | *M | Pyrogallol | 15.41 | A |
| Sodium Capryl Sulfate (14.84) | 15.80 | * | Acetamide (59.00) | 16.03 | C |
| Erythritol | 16.06 | * | Glycerin (42.50) | 16.26 | E0 |
| Formamide (109.0) | 17.82 | E | Ammonia (16.90) | 18.08 | 0 |
| Lactose | 19.50 | * | Water (80.10) | 23.40 | CN |

### References

| NOTE: * = Solubility Parameter value from literature |
|---|
| |

| SOURCE of Physical Data: |
|---|
| A. Aldrich Chemical Co, Catalog 1986 gram |
| B. Beilstein's Index |
| C. Chemical Rubber Handbook of Chemi.& Physics, 42d Ed. (1961-1962) |
| D. Dictionary of Organic Compounds |
| E. Eastman Organic Chemical Bulletin 47, No.1,1975 |
| F. Fisher Scientific Catalog - 1986 |
| G. Group Contribution Method of Hay, Van Krevelen and Feodors. |
| H. HANDBOOK OF SOLUBILITY PARAMETERS, A.F. Barton, Chemical Rubber Publication, 1985 |
| I. INDUSTRIAL WAXES, H. Bennett, Chemical Pub. Co. |
| J. Journal Reference by number 0(x). |
| J1-J.Pharm.Sci.75, (7), 639 |
| J2-Pharm. Acta Helv.,48,549 (1973) |
| J3-Am.Cosmet.Perf.,87,p.85 (1972) |
| K. Kolthof & Elving: TREATISE on ANALYTICAL CHEMISTRY |
| L. Laboratory Determination by: L(1)-Consolbilizer Study |
| L(2)-Solubility Study Unpublished |
| M. Manufacturer's Physical Date by Personal Communication |
| N. Hildebrand & Scott: The Solubility of Nonelectrolytes. Dover Press |
| O. Original published values JSCC 36,319 |
| P. Pharm. Acta Helv.81,(3),95 Antimicrobial Activity and Solubility Parameters-C.V./F.W. |

In the stick compositions according to the invention, the (average) solubility parameter of the totality of the oils present in the presence of linear saturated fatty alcohols having a chain length of at least 8 carbon atoms deviates by at most -0.7 (cal/cm³)^{0.5} or at most +0.7 (cal/cm³)^{0.5}, preferably by at most -0.6. ((cal/cm³)^{0.5} or at most +0.6 (cal/cm³)^{0.5}, particularly preferably by at most -0.4 (cal/cm³)^{0.5} or at most +0.5 (cal/cm³)^{0.5}, and in the presence of water-in-oil emulsifiers which differ from linear saturated fatty alcohols having a chain length of at least 8 carbon atoms, linear saturated fatty alcohols with a chain length of at least 8 carbon atoms being absent, by at most -0.4 (cal/cm³)^{0.5} or at most +0.7 (cal/cm³)^{0.s} preferably by at most -0.3 (cal/cm³)^{0.s} or at most +0.6 (cal/cm³)^{0.5}, particularly preferably by at most -0.2 (cal/cm³)^{0.5} or at most +0.5 (cal/cm³)^{0.5} from the (average) solubility parameter of the water-in-oil emulsifier/water-in-oil emulsifiers. If water-in-oil emulsifier mixtures or oil mixtures are used, the average solubility parameters of the mixture is considered in each case, specifically the arithmetic mean according to the weight fractions of the individual components. Within the scope of the invention, it is also possible for a fraction of up to 20% by weight of the constituent oils that are in a liquid state at 20° C to consist of oils whose solubility parameter deviates by more than -0.4 or -0.7 ((cal/cm³)^{0.5} or by more than +0.7 (cal/cm³)^{0.5}, respectively, from the (average) solubility parameter of the water-in-oil emulsifier (mixture). In a particularly preferred embodiment of the invention, no oils that are in a liquid state at 20° C are present whose solubility parameter deviates by more than ±1.0 (cal/cm³)^{0.5}, preferably by ±0.7 (cal/cm³)^{0.5} and particularly preferably by ±0.5 (cal/cm³)^{0.5} from the (average) solubility parameter of the water-in-oil emulsifier/water-in-oil emulsifiers.

Lipid or wax matrix

The lipid or wax matrix of the stick compositions according to the invention comprises at least one lipid or wax component with a melting point of >50° C, which is neither to be apportioned to the nonionic oil-in-water emulsifiers with an HLB value of more than 7 nor to the nonionic water-in-oil emulsifiers with an HLB value greater than 1.0 and less than or equal to 7.0, which can form liquid crystalline structures with water alone or with water in the presence of a hydrophilic emulsifier.

Generally, waxes are of solid to brittle consistency, coarse to finely crystalline, transparent or translucent to opaque, but not vitreous, and melt above 50° C without decomposition. Just a little above the melting point they are of low viscosity and exhibit a heavily temperature-dependent consistency and solubility.

According to the invention, preference is given, for example, to natural vegetable waxes, e.g., candelilla wax, carnauba wax, Japan wax, sugar cane wax, ouricoury wax, cork wax, sunflower wax, fruit waxes, such as orange waxes, lemon waxes, grapefruit wax, and animal waxes, e.g., beeswax, shellac wax and spermaceti. For the purposes of the invention, it may be particularly preferred to use hydrogenated or hardened waxes: Chemically modified waxes, in particular the hard waxes, such as, for example, montan ester waxes, hydrogenated jojoba waxes and sasol waxes, can also be used as the wax component. Synthetic waxes, which are likewise preferred according to the invention, include, for example, polyalkylene waxes and polyethylene glycol waxes, C₂₀-C₄₀-dialkyl esters of dimer acids, C₃₀-C₅₀-alkyl beeswax and alkyl and alkylaryl esters of dimer fatty acids.

A particularly preferred wax component is chosen from among at least one ester of a saturated monohydric C₁₆-C₆₀-alcohol and a saturated C₈-C₃₆-monocarboxylic acid. According to the invention these also include lactides, the cyclic double esters of α-hydroxycarboxylic acids of the corresponding chain length. Esters of fatty acids and long-chain alcohols have proven particularly advantageous for the composition according to the invention because they impart excellent sensory properties to the antiperspirant preparation and high stability to the stick overall. The esters are composed of saturated, branched or unbranched monocarboxylic acids and saturated, branched or unbranched monohydric alcohols. According to the invention, it is also possible to use esters of aromatic carboxylic acids or hydroxycarboxylic acids (e.g. 12-hydroxystearic acid) and saturated, branched or unbranched alcohols if the wax component has a melting point of >50° C. It is particularly preferred to choose the wax components from the group of esters of saturated, branched or unbranched alkanecarboxylic acids with a chain length from 12 to 24 carbon atoms and the saturated, branched or unbranched alcohols with a chain length from 16 to 50 carbon atoms which have a melting point of >50° C.

In particular, C₁₆₋₃₆-alkyl stearates and C₁₈₋₃₈-alkyl hydroxystearoylstearates, C₂₀₋₄₀-alkyl erucates and cetearyl behenate may be advantageous as the wax component. The wax or the wax components have a melting point of >50° C, preferably >60° C.

A particularly preferred embodiment of the invention comprises a C₂₀-C₄₀-alkyl stearate as wax component. This ester is known under the name Kesterwachs^{®} K82H or Kesterwachs^{®} K80H and is sold by Koster Keunen, Inc. It is the synthetic imitation of the monoester fraction of beeswax and is characterized by its hardness, its oil gelability and its broad compatibility with lipid components. This wax can be used as a stabilizer and as a consistency regulator for W/O and O/W emulsions. Kesterwachs offers the advantage that, even in low. concentrations, it has excellent oil gelability and thus does not make the stick mass too heavy and allows for a velvety release. A further particularly preferred embodiment of the invention comprises cetearyl behenate, i.e. mixtures of cetyl behenate and stearyl behenate, as the wax component. This ester is known under the name Kesterwachs^{®} K62 and is sold by Koster Keunen, Inc.

Further preferred lipid or wax components with a melting point of >50° C are the triglycerides of saturated and optionally hydroxylated C₁₂₋₃₀ fatty acids, such as hardened triglyceride fats (hydrogenated palm oil, hydrogenated coconut foil, hydrogenated castor oil), glyceryl tribehenate (tribehenin) or glyceryl tri-12-hydroxystearate, also synthetic complete esters of fatty acids and glycols or polyols having 2-6 carbon atoms as long as they have a melting point above 50° C, for example, preferably C₁₈-C₃₆ acid triglyceride (Syncrowax^{®} HGL-C).

According to the invention, hydrogenated castor oil, obtainable, e.g., as the commercial product Cutina^{®} HR, is particularly preferred as the wax component.

Further preferred lipid or wax components with a melting point of >50° C are the saturated linear C₁₄-C₃₆-carboxylic acids, in particular myristic acid, palmitic acid, stearic acid and behenic acid, and mixtures of these compounds, e.g., Syncrowax^{®} AW 1C(C₁₈-C₃₆ fatty acids) or Cutina^{®} FS 45 (palmitic and stearic acid).

Preferred deodorant or antiperspirant sticks according to the invention are characterized in that the lipid or wax component a) is chosen from among esters of a saturated, monohydric C₁₆-C₆₀-alkanol and a saturated C₈-C₃₆-monocarboxylic acid, in particular cetyl behenate, stearyl behenate and C₂₀-C₄₀-alkyl stearate, glycerol triesters of saturated linear C₁₂-C₃₀-carboxylic acids, which may be hydroxylated, candelilla wax, carnauba wax, beeswax, saturated linear C₁₄₋C₃₆-carboxylic acids, and mixtures of the above-mentioned substances. Particularly preferred lipid or wax component mixtures a) are chosen from among mixtures of cetylbehenate, stearylbehenate, hardened castor oil, palmitic acid and stearic acid. Further preferred lipid or wax component mixtures a) are chosen from among mixtures of C₂₀-C₄₀-alkyl stearate, hardened castor oil, palmitic acid, and stearic acid.

Further preferred deodorant or antiperspirant sticks according to the invention are characterized in that the total amount of lipid or wax component(s) a) is 4-20% by weight, preferably 8-15% by weight, relative to the overall composition. In a particularly preferred embodiment, the ester/esters of a saturated, monohydric C₁₆-C₆₀-alcohol and a saturated C₈-C₃₆-monocarboxylic acid, which represent(s) the lipid or wax component(s) a), comprise(s) 2-10% by weight, preferably 2-6% by weight, relative to the overall composition.

Oil-in-water emulsifiers

The stick compositions according to the invention comprise at least one nonionic oil-in-water emulsifier with an HLB value of more than 7. These are emulsifiers generally known to the person skilled in the art, as listed, for example, in Kirk-Othmer, "Encyclopedia of Chemical Technology," 3rd edition, 1979, volume 8, pages 913-916. For ethoxylated products, the HLB value is calculated according to the formula HLB=(100-L):5, where L is the weight fraction of the lipophilic groups, i.e., of the fatty alkyl or fatty acyl groups, in the ethylene oxide adducts, expressed in percent by weight.

In selecting nonionic oil-in-water emulsifiers that are suitable according to the invention, it is particularly preferred to use a mixture of nonionic oil-in-water emulsifiers in order to be able to optimally adjust the stability of the stick compositions according to the invention. Here, the individual emulsifier components contribute to the overall HLB value or average HLB value of the oil-in-water emulsifier mixture according to their quantitative proportion of the total amount of the oil-in-water emulsifiers. According to the invention, the average HLB value of the oil-in-water emulsifier mixture is 10-19, preferably 12-18 and particularly preferably 14-17. In order to achieve such average HLB values, oil-in-water emulsifiers from the HLB value ranges 10-14, 14-16 and optionally 16-19 are preferably combined with one another. The oil-in-water emulsifier mixtures can, of course, also comprise nonionic emulsifiers with HLB values in the range from >7-10 and 19-20; such emulsifier mixtures may likewise be preferred according to the invention. However, in another preferred embodiment, the deodorant or antiperspirant sticks according to the invention can also comprise just one oil-in-water emulsifier with an HLB value in the range of 10-19.

Preferred deodorant or antiperspirant sticks according to the invention are characterized in that the nonionic oil-in-water emulsifiers b) are chosen from among ethoxylated C₈-C₂₄-alkanols with, on average, 10-100 mol ethylene oxide per mole, ethoxylated C₈-C₂₄-carboxylic acids with, on average, 10-100 mol ethylene oxide per mole, silicone copolyols with ethylene oxide units or with ethylene oxide and propylene oxide units, alkyl mono- and oligoglycosides having 8 to 22 carbon atoms in the alkyl moiety, and ethoxylated analogs thereof, ethoxylated sterols, partial esters of polyglycerols with 2 to 10 glycerol units and esterified with 1 to 4 saturated or unsaturated, linear or branched, optionally hydroxylated C₈-C₃₀-fatty acid moieties, provided they have an HLB value of more than 7, and mixtures of the above-mentioned substances.

The ethoxylated C₈₋₂₄-alkanols have the formula R¹O{CH₂CH₂O)ₙH, where R¹ is a linear or branched alkyl and/or alkenyl group having 8-24 carbon atoms and n, the average number of ethylene oxide units per molecule, denotes 10-100, preferably 10-30 mol ethylene oxide per 1 mol caprylic alcohol, 2-ethylhexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and brassidyl alcohol and technical-grade mixtures thereof. Adducts of 10-100 mol ethylene oxide onto technical-grade fatty alcohols having 12-18 carbon atoms, such as, for example, coconut, palm, palm kernel or tallow fatty alcohol, are also suitable.

The ethoxylated C₈-C₂₄-carboxylic acids have the formula R¹(OCH₂CH₂)ₙOH where R¹ is a linear or branched saturated or unsaturated acyl group having 8-24 carbon atoms and n, the average number of ethylene oxide units per molecule, denotes 10-100 mol, preferably 10-30 mol, ethylene oxide per 1 mol caprylic acid, 2-ethylhexanoic acid, capric acid, lauric acid, isotridecanoic acid, myristic acid, cetyl acid, palmitoleic acid, stearic acid, isostearic acid, oleic acid, elaidic acid, petroselic acid, arachidic acid, gadoleic acid, behenic acid, erucic acid and brassidic acid, and technical-grade mixtures thereof. Adducts of 10-100 mol of ethylene oxide onto technical-grade fatty acids having 12-18 carbon atoms, such as coconut, palm, palm kernel or tallow fatty acids, are also suitable. Particular preference is given to PEG-50 monostearate, PEG-100 monostearate, PEG-50 monooleate, PEG-100 monooleate, PEG-50 monolaurate and PEG-100 monolaurate.

Particular preference is given to using the C₁₂-C₁₈-alkanols or the C₁₂-C₁₈ carboxylic acids having in each case 10-30 units of ethylene oxide per molecule, and mixtures of these substances, in particular Ceteth-12, Ceteth-20, Ceteth-30, Steareth-12, Steareth-20, Steareth-30, Laureth-12 and Beheneth-20.

In addition, C₈ - C₂₂-alkyl mono- and oligoglycosides are preferably used. C₈ - C₂₂-alkyl mono- and oligoglycosides constitute known standard commercial surfactants and emulsifiers. They are prepared, in particular, by reacting glucose or oligosaccharides with primary alcohols having 8-22 carbon atoms. With regard to the glycoside group, both monoglycosides in which a cyclic sugar group is bonded glycosidically to the fatty alcohol, and also oligomeric glycosides with a degree of oligomerization up to about 8, but preferably of 1-2, more preferably of 1.1-1.4, are suitable. The degree of oligomerization here is a statistical average value, which is based on a homologous distribution such as is customary for such technical- products. Products which are obtainable under the trademark Plantacare^{®} comprise a glucosidically bonded C₈-C₁₆-alkyl group on an aligoglucoside group whose average degree of oligomerization is 1-2, preferably 1.2 - 1.4. Particularly preferred C₈-C₂₂-alkyl mono- and oligoglycosides are chosen from among octyl glucoside, decyl glucoside, lauryl glucoside, palmityl glucoside, cetyl glucoside, isostearyl glucoside, stearyl glucoside, arachidyl glucoside and behenyl glucoside, and mixtures thereof. The acylglucamides derived from glucamine are also suitable as nonionic oil-in-water emulsifiers.

Ethoxylated sterols, in particular, ethoxylated soya sterols, also represent suitable oil-in-water emulsifiers according to the invention. The degree of ethoxylation must be greater than 5, but preferably at least 10, in order to have an HLB value greater than 7. Suitable commercial products are, e.g., PEG-10 Soy Sterol, PEG-16 Soy Sterol and PEG-25 Soy Sterol.

In addition, partial esters of polyglycerols having 2 to 10 glycerol units and esterified with 1 to 4 saturated or unsaturated, linear or branched, optionally hydroxylated C₈-C₃₀-fatty acid groups are preferably used, provided they have an HLB value of more than 7. Particular preference is given to diglycerol monocaprylate, diglycerol monocaprate, diglycerol monolaurate, triglycerol monocaprylate, triglycerol monocaprate, triglycerol monolaurate, tetraglycerol monocaprylate, tetraglycerol monocaprate, tetraglycerol monolaurate, pentaglycerol monocaprylate, pentaglycerol monocaprate, pentaglycerol monolaurate, hexaglycerol monocaprylate, hexaglycerol monocaprate, hexaglycerol monolaurate, hexaglycerol monomyristate, hexaglycerol monostearate, decaglycerol monocaprylate, decaglycerol monocaprate, decaglycerol monolaurate, decaglycerol monomyristate, decaglycerol monoisostearate, decaglycerol monostearate, decaglycerol monooleate, decaglycerol monohydroxystearate, decaglycerol dicaprylate, decaglycerol dicaprate, decaglycerol dilaurate, decaglycerol dimyristate, decaglycerol diisostearate, decaglycerol distearate, decaglycerol dioleate, decaglycerol dihydroxystearate, decaglycerol tricaprylate, decaglycerol tricaprate, decaglycerol trilaurate, decaglycerol trimyristate, decaglycerol triisostearate, decaglycerol tristearate, decaglycerol trioleate and decaglycerol trihydroxystearate.

Particularly preferred deodorant or antiperspirant sticks according to the invention are characterized in that the total amount of nonionic oil-in-water emulsifier b) relative to the overall composition is 0.5-10% by weight, particularly preferably 1-4% by weight and extremely preferably 1.5-3% by weight.

Water-in-oil emulsifiers

The stick compositions according to the invention further comprise at least one nonionic water-in-oil emulsifier with an HLB value greater than 1.0 and less than or equal to 7.0, which can form liquid crystalline structures with water alone or with water in the presence of a hydrophilic emulsifier, as a consistency regulator and/or water binder. The water-in-oil emulsifier(s) mainly contribute(s) to the structure of the lipophilic gel phase which surrounds the dispersed lipid/wax/oil phase, as well as, albeit to a lesser extent, to the structure of the hydrophilic gel phase which stabilizes the aqueous phase. Emulsifiers with an HLB value greater than 1.0 and less than or equal to 7.0 are principally suitable as water-in-oil emulsifiers according to the invention. Some of these emulsifiers are listed, for example, in Kirk-Othmer, "Encyclopedia of Chemical Technology 3rd edition, 1979, volume 8, page 913. For ethoxylated adducts, the HLB value, as already mentioned, can also be calculated.

Preferred water-in-oil emulsifiers are:

- linear saturated alkanols having 12-30 carbon atoms, in particular, having 16-22 carbon atoms, in particular, cetyl alcohol, stearyl alcohol, arachidyl -alcohol, behenyl alcohol and lanolin alcohol or mixtures of these alcohols, as are obtainable in the industrial hydrogenation of vegetable and animal fatty acids;

- esters and, in particular, partial esters of a polyol having 2-6 carbon atoms and linear saturated and unsaturated fatty acids having 12-30, in particular 14-22, carbon atoms, which may be hydroxylated. Such esters or partial esters are, for example, the monoesters and diesters of glycerol or ethylene glycol or the monoesters of propylene glycol with linear saturated and unsaturated C₁₂-C₃₀-carboxylic acids, which may be hydroxylated, in particular those with palmitic acid and stearic acid, the sorbitan mono-, di- or triesters of linear saturated and unsaturated C₁₂-C₃₀-carboxylic acids, which may be hydroxylated, in particular those of myristic acid, palmitic acid, stearic acid or of mixtures of these fatty acids, the mono-, di-, tri- and tetraesters of pentaerythritol having linear saturated fatty acids having 12-30, but especially 14-22, carbon atoms that can be hydroxylated, and the methylglucose mono- and diesters of linear, saturated and unsaturated C₁₂-C₃₀-carboxylic acids, which may be hydroxylated;

- sterols, i.e., steroids which carry a hydroxyl group on the C3 atom of the steroid backbone and are isolated both from animal tissue (zoosterols, e.g., cholesterol, lanosterol) and also from plants (phytosterols, e.g., ergosterol, stigmasterol, sitosterol) and from fungi and yeasts (mycosterols) and which may have low degrees of ethoxylation (1-5 EO);

- alkanols and carboxylic acids having in each case 8-24 carbon atoms, in particular having 16-22 carbon atoms, in the alkyl group and 1-4 ethylene oxide units per molecule, which have an HLB value greater than 1.0 and less than or equal to 7.0,

- glycerol monoethers of saturated and/or unsaturated, branched and/or unbranched alcohols with a chain length of 8-30, in particular 12-18, carbon atoms;

- partial esters of polyglycerols having n=2 to 10 glycerol units and esterified with 1 to 5 saturated or unsaturated, linear or branched, optionally hydroxylated C8-C30-fatty acid groups, provided they have an HLB value of less than or equal to 7,

- and mixtures of the abovementioned substances.

According to the invention, it may be preferred to use just one additional water-in-oil emulsifier. In another preferred embodiment, the compositions according to the invention comprise mixtures, in particular technical-grade mixtures, of at least two additional water-in-oil emulsifiers. A technical-grade mixture is understood, for example, as meaning a commercial product such as Cutina^{®} GMS, which constitutes a mixture of glyceryl monostearate and glyceryl distearate.

Water-in-oil emulsifiers which can be used particularly advantageously are ethylene glycol monostearate, ethyleneglycol distearate, pentaerythrityl monostearate, pentaerythrityl distearate, pentaerythrityl tristearate, pentaerythrityl tetrastearate, stearyl alcohol, cetyl alcohol, glyceryl monostearate, in particular, in the form of the commercial products Cutina® GMS and Cutina® MD (ex Cognis), glyceryl distearate, glyceryl monocaprate, glyceryl monocaprylate, glyceryl monolaurate, glyceryl monomyristate, glyceryl monopalmitate, glyceryl monohydroxystearate, glyceryl monooleate, glyceryl monolanolate, glyceryl dimyristate, glyceryl dipalmitate, glyceryl dioleate, propylene glycol monostearate, propylene glycol monolaurate, sorbitan monocaprylate, sorbitan monolaurate, sorbitan monomyristate, sorbitan monopalmitate, sorbitan monostearate, sorbitan sesquistearate, sorbitan distearate, sorbitan dioleate, sorbitan sesquioleate, sucrose distearate, arachidyl alcohol, behenyl alcohol, polyethylene glycol (2) stearyl ether (Steareth-2), Steareth-5, Oleth-2, diglycerol monostearate, diglycerol monoisostearate, diglycerol monooleate, diglycerol dihydroxystearate, diglycerol distearate, diglycerol dioleate, triglycerol distearate, tetraglycerol monostearate, tetraglycerol distearate, tetraglycerol tristearate, decaglycerol pentastearate, decaglycerol penta-hydroxystearate_{;} decaglycerol pentaisostearate, decaglycerol pentaoleate, soy sterol, PEG-1 soy sterol, PEG-5 soy sterol, PEG-2 monolaurate and PEG-2 monostearate.

With respect to the stability and the hardness of the resulting products also in the large-scale production, ethylene glycol monostearate, ethylene glycol distearate, pentaerythrityl monostearate, pentaerythrityl distearate, pentaerythrityl tristearate, pentaerythrityl tetrastearate, as well as mixtures thereof, proved to be very worthwhile. The commercial products Cutina AGS (INCI: Glycol distearate), Cutina EGMS (INCI: glycol stearate) and Cutina PES (INCI: pentaerythrityl distearate), all ex Cognis, are particularly preferred.

Particularly preferred deodorant or antiperspirant sticks according to the invention are characterized in that the total amount of the nonionic water-in-oil emulsifier(s) c), which can form liquid crystalline structures with water alone or with water in the presence of a hydrophilic emulsifier, relative to the overall composition is 0.1-15% by weight, particularly preferably_{.}0.5-8% by weight and most preferably 1-4% by weight. In addition, amounts of from 2-3% by weight, based on the total weight of the composition, may also be most preferred according to the invention.

The following table contains various oil-in-water emulsifiers and water-in-oil emulsifiers and their HLB values. The HLB values, however, can also be calculated using Griffin's method, as for example in the RÖMPP Chemie Lexikon, specifically the online version of November 2003, and the handbooks from Fiedler, Kirk-Othmer, and Janistyn cited there under the keyword "HLB System." As long as there is conflicting HLB data for a substance found in the literature, the HLB value that comes closest to Griffin's HLB value should be used for the teaching of the invention. If no clear HLB value can be determined this way, the HLB value stated by the manufacturer of the emulsifier should be used for the teaching of the invention. If that is not possible either, then the HLB is determined experimentally.

HLB value chemical designation (from H. Janistyn, Handbuch der Kosmetika und Riechstoffe, Huthig-Verlag Heidelberg, 3. edition, 1978, Volume 1, page 470 and Volume 3, pages 68 - 78))

| | |
|---|---|
| 1 | Triglycerides of saturated fatty acids |
| | Glyceryltrioleate |
| 1.5 | Ethyleneglycol distearate |
| 1.6 | Pure cellin oil |
| 1.8 | Sorbitan trioleate |
| | Glycerol dioleate |
| 2.1 | Sorbitan tristearate |
| 2.4 | Propylene glycol lactostearate |
| 2.7 | Glycerol monooleate |
| | Sorbitol dioleate |
| 2.8 | Glycerol monostearate |
| | Propylene glycol mono-/distearate, non-self-emulsifying |
| 3.0 | Decaglycerol decaoleate |
| | Decaglycerol decastearate |
| | Generol 122 (Rapeseed Sterols) |
| | Sucrose distearate |
| 3.1 | Decaglycerol decaoleate |
| | Glyceryl monoricinoleate |
| | Pentaerythrityl monostearate |
| | Pentaerythrityl sesquioleate |
| 3.2 | Ethyleneglycol monodistearate, non-self-emulsifying |
| | Glycolstearate |
| 3.3 | Glycerol monolaurate |
| 3.4 | Propylene glycol monostearate |
| 3.5 | Ethylene glycol monostearate |
| | Pentaerythrityl monooleate |
| | Polyethylene glycol (100)monooleate |
| 3.6 | Glycerol mono-/dioleate, non-self-emulsifying |
| | Monoethoxylauryl ether |
| 3.7 | Sorbitan sesquioleates (Dehjrmuls SSO) |
| 3.8 | Glycerol monodistearate, non-self-emulsifying |
| | Polyethylene glycol (100) monostearates |
| | Diglycerol sesquioleates |
| | N.N-Dimethylcaproamide |
| | Pentaerythrityl monotallowates |
| | Propylene glycol monolaurate |
| 4.0 | Decaglycerol octaoleate |
| 4.3 | Sorbitan monooleate (Dehymuls SMO) |
| | Diethylene glycol monostearate |
| 4.4 | 1,2-Propylene glycol monodistearate, self-emulsifying |
| 4.5 | Glycerol monostearate palmitate (90%), non-self-emulsifying |
| | Propylene glycol monolaurate |
| 4.7 | Sorbitan monostearate (Dehymuls SMS) |
| | Diethylene glycol monooleate |
| 4.8 | Pentaerythrityl monolaurate |
| 4.9 | Polyoxyethylene(2)oleyl alcohol (Polyoxyethylene(2)oleyl ether) |
| | Polyoxyethylene(2)stearyl alcohol (Polyoxyethylene(2)stearyl ether) |
| 5.0 | Generol 122 E 5 (PEG-5 Soy Sterol) |
| | Polyethylene glycol (100) monoricinoleate |
| | Polyethylene glycol (200) distearate |
| | Polyglyceryl-3-isostearate (e.g. Isolan Gl 34 by Tego) |
| 5.9 | Polyethylene glycol (200) dilaurate |
| 6.0 | Decaglycerol tetraoleate |
| | Polyethylene glycol (100) monolaurates |
| | Polyethylene glycol (200) dioleate |
| 6.3 | Polyethylene glycol (300) dilaurates |
| 6.4 | Glycerol monoricinoleate |
| | Glycerol sorbitan monolaurate |
| 6.5 | Diethylene glycol monolaurate |
| | Sodium stearoyl-2-lactylate |
| 6.7 | Sorbitan monopalmitate |
| 6.8 | Glycerol monococoate |
| | Glycerol monolaurate |
| 7.0 | Polyoxyethylen(2)C10-C14-fatty alcohol ether, Laureth-2 (Dehydol LS 2) |
| | Sucrose distearate |
| 7.2 | Polyethylene glycol (400) dioleate |
| | Sucrose dioleate |
| 7.4 | Polyethylene glycol (100) monolaurate |
| 7.5 | Sucrose dipalmitate |
| 7.6 | Glycerol sorbitan laurate |
| 7.8 | Polyethylene glycol (400) distearates |
| 7.9 | Polyethylene glycol (200) monostearate |
| | Polyoxyethylene (3) tridecyl alcohol |
| 8-8.2 | Polyethylene glycol (400) distearate |
| 8.0 | Polyoxyethylene(3)C10-C14-fatty alcohol, Laureth-3 (Dehydol LS 3) N.N-Dimethyllauramide |
| | Sodium lauroyl- lactylate, sodium lauroyl-2-lactylate |
| | Polyethylene glycol (200) monooleate |
| | Polyethylene glycol (220) monotallowate |
| | Polyethylene glycol (1500) dioleate |
| | Polyoxyethylene (4) oleyl alcohol |
| | Polyoxyethylene (4) stearylcetyl ether |
| 8.2 | Triglycerol monooleate |
| 8.3 | Diethylene glycol monolaurate |
| 8.4 | Polyoxyethylene (4) cetylether |
| | Polyoxyethylene glycol (400) dioleate |
| 8.5 | Sodium caproyl lactylate |
| | Polyethylene glycol (200) monostearate |
| | Sorbitan monooleate |
| 8.6 | Sorbitan monolaurate (Dehymuls SML) |
| | Polyethylene glycol (200) monolaurate |
| 8.8 | Polyoxyethylene (4) myristyl ether |
| | Polyethylene glycol (400) dioleate |
| 8.9 | Nonylphenol, polyoxyethylated with 4 Mol EO |
| 9.0 | Oleth-5 (z. B. Eumulgin O 5) |
| 9.2-9.7 | Polyoxyethylene (4) lauryl alcohol (according to commercial product. e.g. Brij 30, Dehydol LS 4) |
| 9.3 | Polyoxyethylene (4) tridecyl alcohol |
| 9.6 | Polyoxyethylene (4) sorbitan monostearate |
| 9.8 | Polyethylenglycol (200) monolaurate |
| 10-11 | Polyethylene glycol (400) monooleate |
| 10.0 | Didodecyldimethylammoniumchloride |
| 10.0 | Polyethylene glycol (200) monolaurate |
| | Polyethylene glycol (400) dilaurate |
| | Polyethylene glycol (600) dioleate |
| | Polyoxyethylene (4) sorbitan monostearate |
| | Polyoxyethylene (5) sorbitan monooleate |
| 10.2 | Polyoxyethylene (40) sorbitol hexaoleate |
| 10.4 - 10.6 | Polyoxyethylene glycol (600) distearate |
| 10.5 | Polyoxyethylene (20) sorbitan tristearate |
| 10.6 | Sucrose monostearate |
| 10.7 | Sucrose monooleate |
| 11 - 11.4 | Polyethylene glycol (400) monooleate |
| 11.0 | Polyethylene glycol (350) monostearate |
| | Polyethylene glycol (400) monotalleate |
| | Polyoxyethylene glycol (7) monostearate |
| | Polyoxyethylene glycol (8) monooleate |
| | Polyoxyethylene (20) sorbitan trioleate |
| | Polyoxyethylene (6) tridecyl alcohol |
| 11.1 | Polyethylene glycol (400) monostearate |
| 11.2 | Polyoxyethylene (9) monostearate |
| | Sucrose monooleate |
| | Sucrose monostearate |
| 11.4 | Polyoxyethylene (50) sorbitol hexaoleate |
| | Sucrose monotalleate |
| | Sucrose stearate palmitate |
| 11.6 | Polyoxyethylene glycol (400) monoricinoleate |
| 11.7 | Sucrose monomyristeate |
| | Sucrose monopalmitate |
| 12.0 | PEG-10 Soy Sterol (e.g. Generol 122 E 10) |
| | Triethanolamine oleate |
| 12.2-12.3 | Nonylphenol, ethoxylated with 8 Mol EO |
| 12.2 | Sucrose monomyristeate |
| 12.4 | Sucrose monolaurate |
| | Polyoxyethylene (10) oleyl alcohol, polyoxyethylene (10) oleyl ether |
| | Polyoxyethylene (10) stearyl alcohol, polyoxyethylene (10) stearyl ether |
| 12.5 | Polyoxyethylene (10) stearylcetyl ether |
| 12.7 | Polyoxyethylene (8) tridecyl alcohol |
| 12.8 | Polyoxyethylene glycol (400) monolaurate |
| | Sucrose monococoate |
| 12.9 | Polyoxyethylene (10) cetylether. |
| 13 | Glycerol monostearate, ethoxylated (20 Mol EO) |
| 13.0 | Eumulgin O 10 |
| | Eumulgin 286 |
| | Eumulgin B 1 (Ceteareth-12) |
| 13.0 | C12-fat amines, ethoxylated. (5 Mol EO) |
| 13.1 | Nonylphenol, ethoxylated (9.5 Mol EO) |
| 13.2 | Polyethylene glycol (600) monostearate |
| | Polyoxyethylene (16) tallow-oil |
| 13.3 | Polyoxyethylene (4) sorbitan monolaurate |
| 13.5 | Nonylphenol, ethoxylated (10.5 Mol EO) |
| | Polyethylene glycol (600) monooleate |
| 13.7 | Polyoxyethylene (10) tridecyl alcohol |
| | Polyethylene glycol (660) monotallowate |
| | Polyethylene glycol (1500) monostearate |
| | Polyoxyethylene glycol (1500) dioleate |
| 13.9 | Polyethylene glycol (400) monococoate |
| | Polyoxyethylene (9) monolaurate |
| 14-16 | Eumulgin HRE 40 (Ricinus oil, ethoxylated and hydroxylated with 40 EO) |
| 14.0 | Polyoxyethylene (12) lauryl ether |
| | Polyoxyethylene (12) tridecyl alcohol |
| 14.2 | Polyoxyethylene (15) stearyl alcohol |
| 14.3 | Polyoxyethylene (15) stearylcetyl ether |
| 14.4 | Mixture of C12-C15-fatty alcohols with 12 Mol EO |
| 14.5 | Polyoxyethylene (12) lauryl alcohol |
| 14.8 | Polyoxyethylene glycol (600) monolaurate |
| 14.9 -15.2 | Sorbitan monostearate, ethoxylated with 20 EO (e.g. Eumulgin SMS 20) |
| 15 -15.9 | Sorbitan monooleate, ethoxylated with 20 EO (e.g. Eumulgin SMO 20) |
| 15.0 | PEG-20 Glyceryl stearate (e.g. Cutina E 24) |
| | PEG-40 Castor Oil (e.g. Eumulgin RO 40) |
| | Decyl glucoside (Oramix NS 10) |
| | Dodecyl glucoside (Plantaren APG 600) |
| | Dodecyl trimethyl ammonium chloride |
| | Nonylphenol, ethoxyalted with 15 Mol EO |
| | Polyethylene glycol (1000) monostearate |
| | Polyoxyethylene (600) monooleate |
| 15-17 | Eumulgin HRE 60 (Ricinus oil, ethoxylated and hydrated with 60 EO) |
| 15.3 | C12-fat amines, polyoxyethylated with 12 Mol EO |
| | Polyoxyethylene (20) oleyl alcohol, polyoxyethylene (20) oleylether |
| 15.4 | Polyoxyethylene (20) stearylcetylether (z. B. Eumulgin B 2 (Ceteareth-20)) |
| 15.5 | Polyoxyethylene (20) stearyl alcohol |
| 15.6 | Polyoxyethylene glycol (1000)monostearate |
| | Polyoxyethylene (20) sorbitan monopalmitate |
| 15.7 | Polyoxyethylene (20) cetyl ether |
| 15.9 | Disodium triethanolamine distearyl heptaglycol ether sulfosuccinate |
| 16.0 | Nonylphenol ethoxylated with 20 Mol EO |
| | Polyoxyethylene (25) propylene glycol stearate |
| 16 - 16.8 | Polyoxyethylene (30) monostearate |
| 16.3-16.9 | Polyoxyethylene (40) monostearate |
| 16.5 - 16.7 | Polyoxyethylene (20) sorbitan monolaureate (e.g. Eumulgin SML 20) |
| 16.6 | Polyoxyethylene (20) sorbitol |
| 16.7 | C18 fat amines. polyoxyethylated with 5 Mol EO Polyoxyethylene (23) lauryl alcohol |
| 17.0 | Ceteareth-30. z. B. Eumulgin B 3 |
| | Octyl glucoside (Triton CG 110) |
| | Polyoxyethylene (30) glyceryl monolaurate |
| 17.1 | Nonylphenol, ethoxylated with 30 Mol EO |
| 17.4 | Polyoxyethylene (40) stearyl alcohol |

Further preferred stick compounds according to the invention are wherein the total content of nonionic and ionic emulsifiers and/or surfactants with an HLB value above 8 is a maximum of 20% by weight, a preferred maximum of 15% by weight, a particularly preferred maximum of 10% by weight, a particularly preferred maximum of 7% by weight, a further particularly preferred maximum of 4% by weight, and an exceptionally preferred maximum of 3% by weight, preferring respectively to the total compound according to the invention.

Oils

The stick compounds according to the invention contain further at least one oil, which is liquid at 20° C, which does not represent a fragrance component and no essential oil, whereby the (average) solubility parameter of the total of the contained oils in the presence of linear saturated fatty alcohols with a chain length of at least 8 carbon atoms differs by a maximum of -0.7 (cal/cm³)^{0.5} resp. a maximum of +0.7 (cal/cm³)^{0.5}, preferably by a maximum of -0.6 (cal/cm³)^{0.5} resp. a maximum of +0.6 (cal/cm³)^{0.5}, particularly preferably by a maximum of -0.4 (cal/cm³)^{0.5} resp. a maximum of +0.5 (cal/cm³)^{0.5}, and in the presence of water-in-oil emulsifiers, which are different from linear saturated fatty alcohols with a chain length of at least 8 carbon atoms, in the absence of linear saturated fatty alcohols with a chain length of at least 8 carbon atoms by a maximum of -0.4 (cal/cm³)^{0.5} resp. a maximum of +0.7 (cal/cm³)^{0.5}, preferably by a maximum of -0.3 (cal/cm³)^{0.5} resp. a maximum of +0.6 (cal/cm³)^{0.5}, particularly preferably by a maximum of.-0.2 (cal/cm³)^{0.5} resp. a maximum of +0.5 (cal/cm³)^{0.5}, from the (average) solubility parameter of the water-in-oil emulsifier(s). The matching of the used oil(s) with the used water-in-oil emulsifier(s) represents an important parameter of this invention. If the water-in-oil emulsifiers and the oil component(s) do not match each other in their solubility parameter within the required limits, one will get sticks with an unsatisfactory degree of hardness and stability from the point of view of usage.

Preferred oils according to the invention are chosen from branched saturated or unsaturated fatty alcohols with 6-30 carbon atoms. These alcohols are often also known as Guerbet Alcohols, as they are obtained by the Guerbet Reaction. Preferred Guerbet Alcohol oils are hexyldecanol (Eutanol^{®} G 16, Guerbitol^{®} T 16), octyldodecanol (Eutanol^{®} G, Guerbitol^{®} 20), 2-ethylhexylalcohol and the commercial products Guerbitol^{®} 18, Isofol^{®} 12, Isofol^{®} 16, Isofol^{®} 24, Isofol^{®} 36, Isocarb^{®} 12, Isocarb^{®} 16 or Isocarb^{®} 24.

Further preferred oil components are mixtures of Guerbet Alcohols and Guerbet Alcohol esters, for example the commercial product Cetiol^{®} PGL (hexyldecanol and hexyldecyllaurate).

Further preferred oils according to the invention are chosen from the triglycerides of linear or branched, saturated or unsaturated, optionally hydroxylated C₈₋₃₀-fatty acids. The use of natural oils e.g. soya oil, cottonseed oil, sunflower oil, palm oil, palm seed oil, linseed oil, almond oil, castor oil, corn oil, olive oil, rapeseed oil, sesame seed oil, safflower oil, wheat germ oil, peach seed oil and the liquid parts of coconut oil and the like can be particularly suitable. Suitable are however also synthetic triglycerides, in particular capric/caprylic triglycerides, e.g. the commercial products Myritol^{®} 318, Myritol^{®} 331 (Cognis) or Miglyol^{®} 812 (Hüls) with non-branched fatty acid residues as well as glyceryl tri-isostearine and the commercial products Estol^{®} GTEH 3609 (Uniqema) or Myritol^{®} GTEH (Cognis) with branched fatty acid residues.

Further particularly preferred oils according to the invention are chosen from the dicarboxylic acid esters of linear or branched C₂-C₁₀-alkanols, in particular di-isopropyladipate, di-n-butyladipate, di-(2-ethylhexyl)adipate, dioctyl-adipate, diethyl-/di-n-butyl/dioctylsebacate, di-isopropylsebacate, dioctylmalate, dioctylmaleate, dicaprylylmaleate, di-isooctylsuccinate, di-2-ethylheicylsuccinate and di-(2-hexyldecyl)-succinate.

Further particularly preferred oils according to the invention are chosen from the addition products of 1 to 5 propylene oxide units to monohydric or polyhydric C₈₋₂₂-alkanois such as octanol, decanol, decanediol, lauryl alcohol, myristyl alcohol and stearyl alcohol, e.g. PPG-2-myristylether and PPG-3-myristylether (Witconol^{®} APM).

For the use of the oils listed below in the stick compounds according to the invention, care is to be taken that their share in the total oil mixture is only so large that the average solubility parameter of the entire oil mixture, as required by the invention and described above, matches the average solubility parameter of. the water-in-oil emulsifiers. Corresponding oils are chosen from the esters of the linear or branched saturated or unsaturated fatty alcohols with 2 - 30 carbon atoms, which can be hydroxylated. Among these are hexyldecylstearate (Eutanol^{®} G 16 S), hexyldecyllaurate, isodecylneopentanoate, isononylisononanoate, 2-ethylhexylpalmitate (Cegesoft^{®} C 24) and 2-ethylhexylstearate (Cetiol^{®} 868). Similarly, limitedly suitable are isopropylmyristate, isopropylpalmitate, isopropyl-stearate, isopropylisostearate, isopropyloleate, iso-octylstearate, isononylstearate, isocetylstearate, isononylisononanoate, isotridecylisononanoate, cetearyliso-nonanoate, 2-ethylhexyllaurate, 2-ethylhexylisostearate, 2-ethylhexylcocoate, 2-octyldodecylpalmitate, butyloctane acid-2-butyloctanoate, di-isotridecylacetate, n-butylstearate, n-hexyllaurate, n-decyloleate, oleyloleate, oleylerucate, erucyloleate, erucylerucate, ethylenglycoldioleate and -dipalmitate.

Further oils, which are, in consideration of the solubility parameter matching, usable only in small quantities or not usable at all, are chosen from the addition products of at least 6 propylene oxide units to monohydric or polyhydric C₃₋₂₂-alkanols such as butanol, butanediol, myristyl alcohol and stearyl alcohol, e.g. PPG-14-butylether (Ucon Fluid^{®} AP), PPG-9-butylether (Breox^{®} B25), PPG-10-butanediol (Macol^{®} 57) and PPG-15-stearylether (Ariamol^{®} E).

Further oils, which are, in consideration of the solubility parameter matching, usable only in small quantities or not usable at all, are chosen from the C₈-C₂₂-fatty alcohol esters of monovalent or polyvalent C₂-C₇-hydroxycarboxylic acids, in particular the esters of glycol acid, lactic acid, malic acid, tartaric acid, citric acid and salicylic acid. Such esters based on linear C_{14/15}-alkanols, e.g.C₁₂-C₁₅-alkyllactate, and of C_{12/13}-alkanols branched in the 2-position are to be had under the product name of Cosmacol^{®} of the firm Nordmann, Rassmann GmbH & Co, Hamburg, in particular the commercial products Cosmacol^{®} ESI, Cosmacol^{®} EMI and Cosmacol^{®} ETI.

Further oils, which are, in consideration of the solubility parameter matching, usable only in small quantities or not usable at all, are chosen from the symmetric, asymmetric or cyclic esters of the carbonic acid with fatty alcohols, e.g. glycerin carbonate, Dicaprylylcarbonate (Cetiol^{®} CC) or the esters of the DE 197 56 454 A1.

Further oils, which are, in consideration of the solubility parameter matching, usable only in small quantities or not usable at all, are chosen from the esters of dimerized unsaturated C₁₂-C₂₂-fatty acids (dimerized fatty acids) with monohydric linear, branched or cyclic C₂-C₁₈-alkanols or with polyhydric linear or branched C₂-C₆-alkanols.

It can be preferred, according to the invention, to use mixtures of the oils named above.

Preferred deodorant or antiperspirant sticks according to the invention are wherein in the case of oils d), liquid at 20°C, the choice is among dicarboxylic acid esters of linear or branched C₂-C₁₀-alkanols, branched saturated or unsaturated fatty alcohols with 6 - 30 carbon atoms, triglycerides of linear or branched, saturated or unsaturated, optionally hydroxylated C₈₋₃₀-fatty acids, esters of branched saturated or unsaturated fatty alcohols with 2 - 30 carbon atoms with linear or branched saturated or unsaturated fatty acids with 2 - 30 carbon atoms, which can be hydroxylated; addition products of 1 to 5 propylene oxide units to monohydric or polyhydric C₈₋₂₂-alkanols, addition products of at least 6 propylene oxide units to monohydric or polyhydric C₃₋₂₂-alkanols, C₈₋C₂₂-fatty alcohol esters of monovalent or polyvalent C₂₋C₇-hydroxycarboxylic acids, symmetric, asymmetric or cyclic esters of carbonic acid with fatty alcohols, the esters of dimerized unsaturated C₁₂₋C₂₂-fatty acids (dimerized fatty acids) with monohydric linear, branched or cyclic C₂₋C₁₈-alkanols or with polyhydric linear or branched C₂₋C₆-alkanols, as well as mixtures of the substances named above.

Particularly preferred deodorant or antiperspirant sticks are wherein the oil(s), which is/are liquid at 20°C d) is/are contained in a total amount of 3 - 20% by weight, preferred 5 - 14% by weight, particularly preferred 6 - 12% by weight, , relating respectively to the total weight of the combination.

In a further particularly preferred version of the invention, the share of oil(s), whose solubility parameter differs by more than -0.4 resp. -0.7 (cal/cm³)^{0.5} or by more than +0.7 ((cal/cm³)^{0.5} from (the average) solubility parameter of the water-in-oil emulsifier(s), is a maximum of 20% by weight in relation to the total weight of oils, which are liquid at 20° C. In a further particularly preferred version of the invention no such oils are contained, which are liquid at 20° C, the solubility parameter of which differs by more than ± 1.0 (cal/cm³)^{0.5} from (the average) solubility parameter of the water-in-oil emulsifier(s).

Correspondingly less suitable or (depending on the water-in-oil emulsifier used) in fact unsuitable oil components are for example silicon oils and hydrocarbonated oils.

Silicon oils, among which are e.g. dialkyl- and alkylarylsiloxane, such as for example not only cyclopentasiloxane, cyclohexasiloxane, dimethylpolysiloxane and methylphenylpolysiloxane, but also hexamethyldisiloxane, octamethyltrisiloxane and decamethyltetrasiloxane, display solubility parameters in the range of around 5.7 to 6.3 (cal/cm³)^{0.5}, which is a divergence of more than 0.4 (cal/cm³)^{0.5} of the value of most of the water-in-oil emulsifiers used according to the invention.

Natural and synthetic hydrocarbons such as paraffin oils, isohexadecane, isoeicosane, polyisobutene or polydecene, which are available for example under the name Emery^{®} 3004, 3006, 3010 or under the name Ethylflo^{®} from Albemarle or Nexbase^{®} 2004G from Nestle, as well as 1,3-Di-(2-ethylhexyl)-cyclohexane (Cetiol^{®}S) are similarly among the less preferred oil components according to the invention.

The share of silicon oils and/or hydrocarbons is therefore not be more than 20% in relation to the total weight of oils, which are liquid at 20°C, otherwise the sticks according to the invention do not achieve the desired hardness and stability when used. In a particularly preferred version of the invention no silicon oils and/or hydrocarbons, in particular no paraffin- and iso-paraffin hydrocarbons are contained.

Polyols

The stick compounds according to the invention contain further at least one water soluble polyhydric C₂ - C₉-alkanol with 2 - 6 hydroxyl groups and/or at least one water soluble polyethylene glycol with 3 -20 ethylene oxide units, as well as mixtures thereof. These components are preferably chosen from 1,2-propylene glycol, 2-methyl-1,3-propanediol, glycerine, butylene glycols such as 1,2-butylene glycol, 1,3-butylene glycol and 1,4-butylene glycol, pentylene glycols such as 1,2-pentanediol and 1,5-pentanediol, hexanediols, such as 1,6-hexanediol, hexanetriols such as 1,2,6-hexanetriol, 1,2-octanediol, 1,8-octanediol, dipropylene glycol, tripropylene glycol, diglycerine, triglycerine, erythritol, sorbitol, as well as mixtures of the substances named. Suitable water soluble polyethylene glycols are chosen from PEG-3, PEG-4, PEG-6, PEG-7, PEG-8, PEG-9, PEG-10, PEG-12, PEG-14, PEG-16, PEG-18 and PEG-20, as well as mixtures thereof, whereby PEG-3 to PEG-8 are preferred. Also sugar and certain sugar derivatives such as fructose, glucose, maltose, maltitole, mannite, inosite, sucrose, trehalose and xylose are suitable according to the invention.

Preferred deodorant or antiperspirant sticks are wherein at least one water soluble polyhydric C₂ - C₉-alkanol with 2 - 6 hydroxyl groups and/or at least one water soluble polyethylene glycol with 3 - 20 ethylene oxide units is chosen from 1,2-propylene glycol, 2-methyl-1,3-propanediol, glycerine, butylene glycol such as 1,2-butylene glycol, 1,3-butylene glycol and 1,4-butylene glycol, pentylene glycols such as 1,2-pentanediol and 1,5-pentanediol, hexanediols such as 1,6-hexanediol, hexanetriols such as 1,2,6-hexanetriol, 1,2-octanediol, 1,8-octanediol, dipropylene glycol, tripropylene glycol, diglycerine, triglycerine, erythritol, sorbitol as well as mixtures of the substances named above.

Particularly preferred deodorant or antiperspirant sticks according to the inventions are wherein at least one water soluble polyhydric C₂ - C₉-alkanol with 2 - 6 hydroxyl groups and/or at least one water soluble polyethylene glycol with 3 - 20 ethylene oxide units is contained in all in quantities of 3% - 25% by weight, preferably 8% -18% by weight, related respectively to the total composition.

Water

The share of water in the composition according to the invention is 5% to less than 50% by weight, preferably 10% to less than 30% by weight, particularly preferred 15% - 28% by weight, exceptionally preferred 20% - 26% by weight, relative respectively to the total composition.

Ethanol and/or Isopropanol

The total content of ethanol and/or isopropanol in the compositions according to the invention is 1% to 15% by weight, preferably 2% to 10% by weight, particularly preferred 3% to 8% by weight, exceptionally preferred 5% to 7% by weight, relative respectively to the total composition.

The stick compositions according to the invention further contain at least one deodorant and/or antiperspirant active substance.

Deodorant actives

Deodorant actives preferred according to the invention are odor absorbers, de-odorizing ionic exchangers, germ inhibiting agents, pre-biotic components as well as enzyme inhibitors or, particularly preferred, combinations of the named substances.

Silicates serve as odor absorbers, which simultaneously advantageously support the rheological characteristics of the composition according to the invention. Among the particularly advantageous silicates according to the invention are above all layered silicates and among these in particular montmorillonite, kaolinite, illite, beidellite, nontronite, saponite, hectorite, bentonite, smectite and talcum. Further advantageous odor absorbers are for example zeolithes, zincricinoleate, cyclodextrines, and certain metallic oxides such as e.g. aluminum oxide as well as chlorophyll. They are preferably used in a quantity of 0.1% -10% by weight, particularly preferred 0.5% - 7% by weight and exceptionally preferred 1% - 5% by weight, relating respectively to the total composition.

By germ inhibiting or anti-microbial substances in the context of the invention are meant such substances, which reduce the number resp. growth of odor engendering germs inhabiting the skin. Among these germs are among others various species of the group of staphylococci, and of the groups coryne bacteria, anaerococci and micrococci.

Preferred as germ inhibiting or anti-microbial substances according to the invention are in particular organo-halogen compounds as well as organo halogenides, quaternary ammonium compounds, a series of vegetable extracts and zinc compounds. Among these are among others triclosan, chlorhexidine and chlorhexidine gluconate, 3,4,4'-trichlorcarbanilide, bromchlorophene, dichlorophene, chlorothymol, chloroxylenol, hexachlorophene, dichloro-m-xylenol, dequalinium chloride, domiphene bromide, ammonium phenolsulfonate, benzalconium halogenides, benzalconium cetylphosphate, benzalconium saccharinate, benzethonium chloride, cetylpyridinium chloride, laurylpyridinium chloride, laurylisoquinolinium bromide, methylbenzedonium chloride. Further usable are phenol, phenoxyethanol, dinatrium-dihydroxy-ethylsulfo-succinyl-undecylenate, sodium bicarbonate, zinc lactate, sodium phenolsulfonate and zinc phenolsulfonate, ketoglutar acid, terpen alcohols such as e.g. farnesol, chlorophylline copper complexes, α-mono-alkylglycerine ethers with a branched or linear saturated or unsaturated, optionally hydroxylated C₆ - C₂₂-alkyl residue, particularly preferred α-(2-ethylhexyl) glycerine ethers, commercially available as Sensiva^{®} SC 50 (ex Schülke & Mayr), carboxylic acid esters of the mono-, di- and tri glycerines (e.g. glycerine monolaurate, diglycerine monocaprinate), lantibiotics as well as vegetable extracts (e.g. green tea and parts of linden blossom oil).

Further preferred deodorant substances are chosen from so-called prebiotic components, by which such components in the context of the invention are meant that inhibit only or at least preponderantly the odor engendering germs of the skin microflora, but not the desired, i.e. the non-odor engendering germs, which belong to healthy skin flora. Explicitly included here are the substances that are described in the publications DE 10333245 and DE 10 2004 011 968 as prebiotic in effect; among these are coniferous extracts, in particular of the group of the pinaceae, and vegetable extracts of the group of Sapindaceae, Araliaceae, Lamiaceae and Saxifragaceae, in particular extracts of *Picea spp., Paullinia sp., Panax sp., Lamium album* or *Ribes nigrum* as well as mixtures of these substances.

Further preferred deodorant substances are chosen from germ inhibiting perfumed oils and Deosafe^{®} perfumed oils, which are available from the firm Symrise, formerly Haarmann and Reimer.

Among the enzyme inhibitors are substances that inhibit the enzymes responsible for decomposition of sweat, in particular arylsulfatase, β-glucuronidase, aminoacylase, esterases, lipases and/or lipoxigenase e.g. trialkylcitric acid esters, in particular triethyl-citrate, or zinc glycinate.

Preferred deodorant or antiperspirant sticks according to the invention are wherein at least one deodorant active substance is chosen from arylsulfatase inhibitors, β-Glucuronidase inhibitors, aminoacylase inhibitors, esterase inhibitors, lipase inhibitors and lipoxigenase inhibitors, α-monoalkylglycerine ethers with one branched or linear saturated or unsaturated, optionally hydroxylated C₆ - C₂₂-alkyl residue, in particular α-(2-ethylhexyl) glycerine ether, phenoxyethanol, germ inhibiting perfumed oils, Deosafe^{®} perfumed oils, prebiotic components, trialkyl citric acid esters, in particular triethylcitrate, substances that reduce the number of odor generating skin bacteria of the group of staphylococci, coryne bacteria, anaerococci and micrococci resp. inhibit their growth, zinc compounds, in particular zinc phenolsulfonate and zinc ricinoleate, organo-halogen compounds, in particular triclosan, chlorhexidine, chlorhexidine gluconate and benzalconium halogenides, quaternary ammonium compounds, in particular cetylpyridinium-chloride, odor absorbers, in particular silicates and zeolithes, sodium bicarbonate, lantibiotics, as well as mixtures of the substances mentioned above.

Further preferred deodorant or antiperspirant sticks according to the invention are wherein at least one deodorant active substance is contained in a total quantity of 0.1% - 10% by weight, preferably 0.2% - 7% by weight, particularly preferred 0.3% - 5% by weight and exceptionally preferred 0.4% - 10% by weight, related to the total weight of the active substance in the total composition.

Antiperspirant active substances

Preferred deodorant or antiperspirant sticks according to the invention are wherein at least one antiperspirant active substance, chosen from the water soluble astringent inorganic and organic salts of aluminum, zirconium and zinc resp. desired mixtures of these salts is contained. Particularly preferred antiperspirant active substances are chosen from aluminum chlorhydrates, in particular the aluminum chlorhydrates with the general formula [Al₂(OH)ₛCl. 2-3 H₂O]ₙ that can exist in the non-active or the active (depolymerized) form, further aluminum sesquichlorhydrate, aluminum chlorhydrex-propylene glycol (PG) or - polyethylene glycol (PEG), aluminum sesquichlorhydrex-PG or -PEG, aluminum-PG-dichlorhydrex or aluminum-PEG-dichlorhydrex, aluminum hydroxide, further chosen from the aluminum zirconium chlorhydrates, such as aluminum zirconium trichlorhydrate, aluminum zirconium tetrachlorhydrate, aluminum zirconium penta-chlorohydrate, aluminum zirconium octachlorohydrate, the aluminum-zirconium-chlorohydrate-glycine complexes such as aluminum zirconium trichlorohydrex-glycine, aluminum zirconium tetrachlorohydrexglycine, aluminum zirconium pentachlorohydrexglycine, aluminum zirconium octachlorohydrexglycine, potassium aluminum sulfate (KAl(SO₄)₂ - 12 H₂O, Alaun), aluminum undecylenoyl collagen amino acid, sodium aluminum lactate + aluminum sulfate, sodium aluminum chlorohydroxylactate, aluminum bromhydrate, aluminum chloride, the complexes of zinc and sodium salts, the complexes of lanthan and cer, the aluminum salts of lipo amino acids, aluminum sulfate, aluminum lactate, aluminum chlorohydroxyallantoinate, sodium-aluminum-chlorhydroxylactate, zinc chloride, zinc sulfocarbolate, zinc sulfate and zirconium chlorohydrate. In the context of the invention by water solubility a solubility of at least 5% by weight at 20 °C is to be understood, that means that quantities of at least 5 g of the antiperspirant active substances are soluble in 95 g water at 20 °C. The antiperspirant active substances can be used as aqueous solutions.

Particularly preferred deodorant or antiperspirant sticks according to the invention are wherein at least one antiperspirant active substance is contained in a quantity of 3% - 27% by weight, preferably 5% - 22% by weight and in particular 10% - 20% by weight, related to the total weight of the active substance in the total composition. In a particularly preferable version the composition contains the combination of an astringent aluminum salt, in particular aluminum chlorohydrate, which for example is sold in powder form as Micro Dry^{®} Ultrafine from Reheis, in the form of an aqueous solution as Locron^{®} L from Clariant, as Chlorhydrol^{®}, as well as in active form as Reach^{®} 501 from Reheis. A certain aluminum sesquichlorohydrate from Reheis is offered under the name Reach^{®} 301, which is similarly particularly preferred. Also the use of aluminum-zirconium-tetrachlorohydrex-glycine complexes, which for example are commercially available under the name Rezal^{®} 36G, can be particularly preferred in the context of the invention.

The stick compositions according to the invention can contain, in a further particularly preferred version, at least one deodorant as well as also at least one antiperspirant active substance.

Low melting point lipid or wax components

Particularty preferred deodorant or antiperspirant sticks are wherein at least one lipid or wax component with a melting point in the range of 25° - < 50° C, chosen from coconut fatty acid glycerine mono-, di- and tri- esters, butyrospermum parkii (Shea Butter) and esters of saturated, monohydric C₈-C₁₈ alcohols with saturated C₁₂-C₁₈ monocarboxylic acids, as well as mixtures of these substances, is contained. These low melting point lipid or wax components enable an optimizing of the consistency. of the product and a minimizing of the visible residues on the skin. Particularly preferred are commercial products with the INCl designation Cocoglycerides, in particular the commercial products Novata^{®} (ex Cognis), particularly preferred Novata^{®} AB, a mixture of C₁₂-Cₗ₈-mono-, di- and triglycerides, which melts in the range of 30 - 32° C, as well as the products of the Softisan line (Sasol Germany GmbH) with the INCI designation Hydrogenated Cocoglycerides, in particular Softisan 100, 133, 134, 138, 142. Further preferable esters of saturated, monohydric C₁₂-C₁₈ alcohols with saturated C₁₂-C₁₈ monocarboxylic acids are stearyl laurate, cetearyl stearate (e.g. Crodamol^{®} CSS), cetylpalmitate (e.g. Cutina^{®} CP) and myristylmyristate (e.g. Cetiol^{®} MM).

Further particularly preferred deodorant or antiperspirant sticks are wherein at least one lipid or wax component is contained with a melting point in the range of 25° - < 50°C in quantities of 0.01 % to 20% by weight, a preferable 3% - 20% by weight, a particularly preferred 5% - 18% by weight and an exceptionally preferred 6% - 15% by weight, related to the total composition.

Fillers

Particularly preferred deodorant or antiperspirant sticks according to the invention are wherein they contain further at least one solid, water-insoluble, particulate filler for the improvement of the consistency of the stick and the sensory characteristics. In an exceptionally preferred version this filler is chosen from starches, which may be modified optionally (e.g. of corn, rice, potatoes) and starch derivatives, which are pre-gelatinized if desired, in particular aluminium starch octenyl succinate, available under the name DRY FLO^{®}, and similar starch derivatives, e. g. sodium starch octenyl succinate, cellulose and cellulose derivatives, silicon dioxide; silicic acids, e.g. Aerosil^{®}-types, spherical polyalkylsesquisiloxan particles (in particular Aerosil^{®} R972 and Aerosil^{®} 200V from Degussa), silicic gels or silica, talcum, kaolin, clays, e.g. bentonites, magnesium aluminum silicates, bornitride, lactoglobuline derivatives, e.g. sodiUM-C₈₋₁₆ isoalkylsuccinyl lactoglobulin sulfonate, available from Brooks Industries as the commercial product Biopol^{®} OE, glass powders, polymer powders, in particular of polyolefins, polycarbonates, polyurethanes, polyamides, e.g. nylon, polyesters, polystyrenes, polyacrylates, (meth)acrylate- or (meth)acrylate-vinylidene-copolymers, which can be cross-linked, or silicones, as well as mixtures of these substances.

Polymer powders based on a polymethacrylate-copolymer are available, for example as the commercial product Polytrap^{®} 6603 (Dow Corning). Other polymer powders, e.g: based on polyamides, are available under the name Orgasol^{®} 1002 (polyamide-6) and Orgasol^{®} 2002 (polyamide-12) from Elf Atochem. Further polymer powders that are suitable for the purposes of the invention are, for example, polymethacrylate (Micropearl^{®} M from SEPPIC or Plastic Powder A from NIKKOL), styrene-divinylbenzene-copolymers (Plastic Powder FP from NIKKOL), polyethylene- and polypropylene powders (ACCUREL^{®} EP 400 from AKZO) or also silicone polymers (silicone powder X2-1605 from Dow Corning).

Particularly preferred deodorant or antiperspirant sticks according to the invention are wherein they contain at least one solid, water-insoluble, particulate filler in a total quantity of 0.01 % to 30% by weight, preferably 5% - 20% by weight, a particularly preferred 8% - 15% by weight, relating respectively. to the total composition.

Scents

Particularly preferred deodorant or antiperspirant sticks according to the invention are wherein further at least one scent component is contained. As scent components perfumes, perfumed oils or perfume oil parts can be used. Perfumed oils resp. scents can be in the context of the invention individual compounds of odorous substances, e.g. the synthetic products of the type of esters, ethers, aldehydes, ketones, alcohols and hydrocarbons. Odorous substance compounds of the type of esters are e.g. benzyl acetate, phenoxyethylisobutyrate, p-tert.-butylcyclohexylacetate, linalyl acetate, dimethylbenzylcarbinyl acetate (DMBCA), phenylethylacetate, benzyl acetate, ethylmethylphenylglycinate, allylcyclohexylpropionate, styr allyl propionate, benzyl salicylate, cyclohexylsalicylate, flora mate , melusate and jasmecyclate. Among the ethers are for example benzylethylether and ambroxan, among the aldehydes e.g. the linear alkanales with 8 - 18 C atoms, citral, citronellal, citronellyloxy-acetaldehyde, cyclamenaldehyde, lilial and bourgeonal, among the ketones e.g. the jonones, alpha-Isomethylionone and methylcedrylketone, among the alcohols anethol, citronellol, eugenol, geraniol, linalool, phenylethylalcohol and terpineol, among the hydrocarbons primarily the terpenes such as lemons and pines. Mixtures of various odorous substances are preferably used, which orchestrate a suitable perfume blend.

Such perfumed oils can also contain natural mixtures of odorous substances, such as are available from vegetable sources, e.g. pine, citrus, jasmine, patchouli, rose or ylang-ylang oil. Similarly suitable are muscatel salve oil, chamomile oil, carnation oil, melissa oil, mint oil, cinnamon leaf oil, linden blossom oil, juniper berry oil, vetiver oil, olibanum oil, galbanum oil, labdanum oil, as well as orange blossom oil, neroli oil, orange peel oil and sandalwood oil.

In order to be perceived by the senses, an odorous substance must be evanescent, whereby along with the nature of the functional groups and the structure of the chemical compound, the molar mass also plays an important role. For this reason most of the odorous substances possess molar masses up to approximately 200 Daltons, while molar masses of 300 Daltons and above represent more an exception. On the basis of the differing evanescence of odorous substances, the odor of a perfume resp. scent composed of several odorous substances changes during the vaporizing process, whereby the odorous impression are divided into the "top note," "average note resp. body" and the "end note resp. dry out." Since the sensory perception depends to a large extent on the intensity of the odor, the top note of a perfume resp. scent does not consist alone of easily evanescent compounds, while the end note consists for the most part of less evanescent i.e. more enduring odorous substances. In the composition of perfume more easily evanescent odorous substances can be bound for example to certain fixatives, through which their too rapid vaporization is hindered. In the following classification of odorous substances in "more easily evanescent" resp. "enduring" odorous substances, nothing is said about the impression of the odor and about whether the corresponding odorous substance is perceived as top or average note.

Enduring odorous substances that are usable in the context of the present invention are, for example, the etherizing oils such as angelica radix oil, anise oil, arnica blossom oil, basil oil, bay oil, bergamot oil, champak blossom oil, fir oil, turpentine oil, elemi oil, eucalyptus oil, fennel oil, pine needle oil, galbanum oil, geranium oil, ginger grass oil, guaiac wood oil, balsam of gurjun oil, helichrysum/chasteweed oil, ho oil, ginger oil, iris oil, cajeput oil, calmus oil, chamomile oil, camphor oil, canaga oil, cardamom oil, cassia oil, fir cone oil, balsam of kopaiva oil, coriander oil, crisped mint oil, caraway oil, cumin oil, lavender oil, lemon grass oil, lime oil, tangerine oil, melissa oil, ambrette oil, myrrh oil, carnation oil, neroli oil, niaouli oil, oliban oil, orange oil, origanum oil, palmarosa oil, patchouli oil, peru balsam oil, petit grain oil, pepper mint oil, pimento oil, pine oil, rose oil, rosemary oil, sandal wood oil, celery oil, lavender oil, star anise oil, turpentine oil, thuja oil, thyme oil, verbena oil, vetiver oil, juniper berry oil, absinthe oil, winter green oil, ylang-ylang oil, hyssop oil, cinnamon oil, cinnamon leaf oil, citronella oil, lemon oil and cypress oil.

But also the less vaporizing resp. solid odorous substances of natural or synthetic origin can be used in the context of the present invention as enduring odorous substances resp. mixtures of odorous substances, i.e. scents. Among these compounds are the compounds named in the following as well as mixtures thereof: ambrettolide, a-amylzimtaldehyde, anethol, anisaldehyde, anise alcohol, anisol, anthranil acid methylester, acetophenone, benzylacetone, `benzaldehyde, benzoe acid ethylester, benzophenone, benzyl alcohol, benzylacetate, benzylbenzoate, benzylformiate, benzylvalerianate, borneol, bornylacetate, α-bromstyrol, n-decylaldehyde, n-dodecylaldehyde, eugenol, eugenolmethylether, eucalyptol, farnesol, fenchon, fenchylacetate, geranylacetate, geranylformiate, heliotropine, heptincarboxylic acid methylester, heptaldehyde, hydrochinon-dimethylether, hydroxy cinnamic aldehyde, hydroxy cinnamic alcohol, indol, iron, isoeugenol, isoeugenolmethylether, isosafrol, jasmon, camphor, carvacrol, carvon, p-cresolmethylether, cumarin, p-methoxyacetophenone, methyl-n-amylketone, methylanthranil acid methylester, p-methylacetophenone, methylchavikol, p-methylchinoline, methyl-β-naphthylketone, methyl-n-nonylacet-aldehyde, methyl-n-nonylketone, muskon, β-naphthotethytether, β-naphtholmethylether, nerol, nitrobenzol, n-nonylaldehyde, nonyl alcohol, n-octylaldehyde, p-oxy-acetophenone, pentadekanolide, β-phenytethy) alcohol, phenylacetaldehyde-dimethyacetal, phenyl acetic acid, pulegone, safrol, salicylic acid isoamylester, salicylic acid methylester, salicylic acid hexylester, salicylic acid cyclohexylester, santalol, skatol, terpineol, thyme, thymol, γ-undelactone, vanilline, veratrumaldehyde, cinnamic aldehyde, cinnamic alcohol, cinnamic acid, cinnamic acid ethylester, cinnamic acid benzylester.

Among the more evanescent odorous substances are the more easily vaporizing odorous substances of natural or synthetic origin, which can be used alone or in mixtures. Examples of more easily evanescent odorous substances are alkylisothiocyanates (alkyl mustard oils), butandion, lemons, linalool, linaylacetate and -propionate, menthol, menthon, methyl-n-heptenone, phellandrene, phenylacetaldehyde, terpinylacetate, citral, citronellal.

Particularly preferred deodorant or antiperspirant sticks according to the invention are wherein at least one scent component is contained in a total quantity of the 0.00001 % to 4 % by weight, preferably 0.5% - 2 % by weight, relating respectively to the total compositions.

Penetration force values

In a further particularly preferred version the stick compositions according to the invention are characterized by a penetration force value in the range of 150 - 800 gram force (g-force), preferably in the range of 250 - 600 gram force (g-force), particularly preferred in the range of 300 - 520 gram force (g-force), and extremely preferred 340 - 500 gram force (g-force), at a penetration depth of 5.000 mm. The penetration force value represents a measure for the hardness, of a stick (or even of a solid cream composition) and states with which maximum force a defined measuring probe, here a cone of stainless steel with 45° (Model TA 15), is thrust vertically (axially) into the antiperspirant mass to be measured up to a penetration depth of 5.000 mm (five point zero zero zero mm) with a penetrative speed of 2 mm/second. The measurement of the penetration force value is carried out with the TA-XT2i Texture Analyzer of the firm Stable Micro Systems (Vienna Court, Lammas Road, Godalming, Surrey GU7 1YL, England). The maximum force is shown in gram force (g-force). Here lower values characterize a softer composition; harder compositions have a higher penetration force value. Cream-type compositions are often measured with a penetration depth of 10.000 mm (ten point zero zero zero mm), in order to obtain more exact values. This depth of penetration usually cannot be measured with the harder stick masses since in this case the stick mass often begins to break. A doubling of the penetration depth means approximately a trebling up to a quadrupling of the measuring value of the maximum force. The measurements take place at ambient conditions of 30° C and 50% relative humidity; the specimen temperature is 23°C. The measurements take place preferably 3 days and/or 4 weeks after the manufacture of the stick according to the invention.

The antiperspirant creams published in DE 199 62 878 A1 and DE 199 62 881 A1 display penetration force values of 9 - 15 gram force (g-force) under the measuring conditions named here.

Electrical resistance

The state-of-the-art water-containing sticks are almost exclusively in the form of water-in-oil emulsions or emulsions with the aqueous phase as the dispersed phase. In order to delimit the sticks according to the invention clearly and unequivocally from the state of technology, the measurement of the electrical resistance serves as a quick and reliable test, as is usual in the examinations of emulsions. An oil-in-water system due to the continuous water phase displays a higher electrical conductivity and corresponding to this a lower electrical resistance than a water-in-oil system. In a further particularly preferred version, electrical resistance of a maximum of 300 kΩ characterizes the stick compositions according to the invention. Preferable is an electrical resistance of a maximum of 100 kΩ, particularly preferred of a maximum of 80 kΩ. The resistance is measured with a Voltcraft model VC820 multimeter with an automatic measuring range conversion (0-400 Ω / 40MΩ (± 1% + 2dgt)) and two micro-tipped measuring antennae 1.0 mm of stainless steel. The distance between the electrodes is fixed through a millimeter gauge. The measurement is carried out at room temperature (22°C). For this the micro-tipped electrodes are fixed parallel at a distance of 27.0 mm on the millimeter gauge and are connected to the resistance-measuring device. The measurement of the electrical resistance takes place directly on the water-containing antiperspirant sticks. For this the usually curved surface of the antiperspirant sticks is cleared away with a knife to the extent that a flat cross section results. Immediately following this the measuring electrodes are stuck vertically approximately 5 mm into the stick mass. The measured values of the electrical resistance are read off after 30 seconds. The cleaning of the measuring electrodes takes place with a cellulose cloth soaked in alcohol. Under the named measuring conditions tap water displays an electrical resistance of 250 kΩ, a 20% by weight aqueous aluminum chlorohydrate solution 3 kΩ and fully desalinated water 1.7 MΩ.

Further Active Ingredients

Particularly preferred deodorant or antiperspirant sticks according to the invention are wherein further pigments, e.g. titanium dioxide, are contained. The pigment content supports the cosmetic acceptance of the preparation on the part of the user. Further particularly preferred deodorant or antiperspirant sticks according to the invention are wherein they contain the usual component parts of cosmetic preparations, e.g. coloring agents, nanospheres, preservatives and photo-protective substances, anti-oxidants, enzymes as well as conditioners. These are contained in particularly preferred deodorant or antiperspirant sticks preferably in a quantity of 0.001% - 20% by weight.

Product stabilization

Particularly preferred deodorant or antiperspirant sticks are wherein they contain at least one radical-capturing substance for the purposes of product stabilization, particularly preferred being a substance with the INCI designation Tris (tetramethyl-hydroxy-piperidinol) citrate, which for example is available under the commercial name Tinogard Q of the firm Ciba. Tris (tetramethylhydroxy-piperidinol) citrate is contained preferably in quantities of 0.01% - 0.1%, particularly preferred being a 0.025% - 0.05% by weight, relating to the total weight of the composition according to the invention.

Further particularly preferred deodorant or antiperspirant sticks are wherein they contain at least one UV filter. Here the UV filters are preferably chosen from benzotriazole derivatives, in particular 2,2'-methylene-bis-(6-(2H-benzotriazole-2-yl)-4-(1,1,3,3-tetramethylbutyl)-phenol) [Tinosorb M (Ciba)], 2,2'-methyl-bis-[6(2H-benzotriazole-2-yl)-4-(methyl)phenol] (MIXXIM BB/200 of the firm Fairmount Chemical), 2-(2'-hydroxy-3',5'-di-t-amylphenyl)benzotriazole (CAS-No.: 025973-551), 2-(2'-hydroxy-5'-octylphenyl)benzotriazole (CAS-No. 003147-75-9), 2-(2'-hydroxy-5'-methylphenyl)benzotriazole (CAS-No. 2440-22-4), 2-(2H-benzotriazole-2-yl)-4-methyl-6-[2-methyl-3-[1,3,3,3-tetramethyl-1-((trimethylsilyl)-oxy]disiloxanyl)propyl]-phenol (CAS-No.: 155633-54-8) with the INCI designation drometrizole trisiloxane, 2,4-bis-{[4-(2-ethyl-hexyloxy)-2-hydroxy]-phenyl}-6-(4-methoxyphenyl)-1,3,5-triazine (INCI: bis-ethylhexyloxyphenol methoxyphenyl triazine or also aniso triazine, available as Tinosorb^{®} S from CIBA), 2,4-bis-{[4-(3-sulfonato)-2-hydroxy-propyloxy)-2-hydroxy]-phenyl}-6-(4-methoxyphenyl)-1,3,5-triazine-sodium salt, 2,4-bis-{[4-(3-(2-propyloxy)-2-hydroxy-propyloxy)-2-hydroxy]-phenyl}-6-(4-methoxyphenyl)-1,3,5-triazine, 2,4-bis-{[4-(2-ethyl-hexyloxy)-2-hydroxy]-phenyl}-6-[4-(2-methoxyethylcarboxyl)-phenylamino]-1,3,5-triazine, 2,4-bis-{[4-(3-(2-propyloxy)-2-hydroxy-propyloxy)-2-hydroxyJ-phenyl}-6-[4-(ethylcarb-oxyl)-phenylamino]-1,3,5-triazine, 2,4-bis-{[4-(2-ethylhexyloxy)-2-hydroxy]-phenyl}-6-(1-methy)-pyrrol-2-yl)-1,3,5-triazin, 2,4-bis-{[4-tris(trimethylsiloxy-silyl-propyloxy)-2-hydroxy]-pheny}-6-(4-methoxyphenyl)-1,3,5-triazine, 2,4-bis-{[4-(2-methylpropenyloxy)-2-hydroxy]-phenyl}-6-(4-methoxyphenyl)-1,3,5-triazine, 2,4-bis-{[4-(1',1',1',3',5',5',5'-heptamethylsiloxy-2-methyl-propyloxy)-2-hydroxy]-phenyl}-6-(4-methoxyphenyl)-1,3,5-triazine, as well as mixtures of the components named above. Further the addition of water-soluble UV filters is preferable. Preferred water soluble UV filters are 2-phenylbenzimidazole-5-sulfonic acid, phenylene-1,4-bis-(2-benzimidazyl)-3,3'-5,5'-tetrasulfonic acid and their alkali-, earth alkali-, ammonium-, alkylammonium-, alkanolammonium- and glucammoni-um salts, in particular the sulfonic acid itself with the INCI designation of phenyl-benzimidazole sulfonic acid (CAS.-No. 27503-81-7), which for example is available under the commercial name of Eusolex 232 with Merck or under Neo Heliopan Hydro with Symrise, and the phenylene-1,4-bis-(2-benzimidazyl)-3,3'-5,5'-tetrasulfonic acid-bis-sodium salt with the INCI designation disodium phenyl-dibenzimidazole tetrasulfonate (CAS-No.: 180898-37-7), which is for example available under the commercial name of Neo Heliopan AP with Symrise, sulfonic acid derivatives of benzophenonene, preferably 2-hydroxy-4-methoxybenzophenone-5-sulfonic acid and its salts and sulfonic acid derivatives of the 3-benzyli-dencamphor, such as e.g.4-(2-oxo-3-bornylidenmethyl)benzol sulfonic acid.

Further particularly preferred . deodorant or antiperspirant sticks are wherein they contain the radical capturing agent tris (tetra-methylhydroxy-piperidinol) citrate and the UV filter bumetrizole for the purposes of product stabilization. Bumetrizole is contained preferably in quantities of 0.01% - 0.1%,. particularly preferred being 0.025% - 0.05% by weight, relating to the total weight of the composition according to the invention.

Further particularly preferred deodorant or antiperspirant sticks are wherein they contain at least one complexing substance for the purposes of product stabilization. As a complexing substance particularly preferred is ethylenediaminetetra-acetic acid (EDTA) and its sodium salts, such as are for example available under the commercial name of Trilon B of the firm BASF, further nitrilotri-acetic acid (NTA) and its sodium salts, β-alanindi-acetic acid and its salts and phosphonic acids and their salts. The complexing substance, at least one in number, is preferably contained in a total weight of 0.01 % - 0.5% by weight, particularly preferred in a 0.08% - 0.2% by weight, relating to the total weight of the composition according to the invention.

Further extraordinarily preferred deodorant or antiperspirant sticks according to the invention are wherein they contain at least one radical-capturing substance and at least one substance chosen from UV filters and complexing substances.

Further extraordinarily preferred deodorant or antiperspirant sticks according to the invention are wherein they contain at least one radical-capturing substance, at least one UV filter and at least one complexing substance.

Further extraordinarily preferable deodorant or antiperspirant sticks according to the invention are characterized by the fact that they contain tris(tetramethylhydroxy-piperidinol) citrate, Bumetrizole and ethylenediaminetetraacetic acid, the latter optionally as sodium salt.

Hair growth inhibitors

Further particularly preferred deodorant or antiperspirant sticks are wherein they contain at least one hair-growth inhibiting substance. Suitable substances that inhibit hair-growth are in particular chosen from eflornithine, substance combinations of soya protein hydrolysate, urea, menthol, salicylic acid and extracts of hypericum perforatum, hamamelis virginiana, arnica montana and the bark of Salix alba, such as is for example contained in the raw material "Pilinhib^{®} Veg LS 9109" of Laboratoires Sérobiologiques with the INCI declaration "Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid," further substance combinations of extracts of Epilobium angustifolium, the seeds of Cucurbita pepo (pumpkin) and the fruits of Serenoa serrulata, such as are for example and preferably contained in the raw material "ARP 100" of Greentech S.A./Rahn with the INCI declaration "Water, Alcohol, Serenoa Serrulata Fruit Extract, Epilobium Angustifolium Extract, Cucurbita Pepo (pumpkin) Seed Extract," further substance combinations of xylitol and the extracts of Citrus medica limonum (lemon) fruit, Carica papaya (papaya) and olive leaves, such as are contained for example and preferably in the raw material "Xyleine" from Impag/Seporga with the INCI declaration "Xylitol and Citrus Medica Limonum (Lemon) Fruit Extract and Carica Papaya (Papaya) Fruit Extract and Olea europaea (olive) leaf extract," further substance combinations of Humulus lupulus, Viscum album, Salvia officinalis, Carica papaya and Thuya occidentalis, such as are contained for example and preferably in the raw material Plantafluid Complex AH of the firm Plantapharm with the INCI declaration "Aqua, Propylene Glycol, Humulus Lupulus, Viscum Album, Salvia Officinalis, Carica Papaya, Thuya Occidentalis," as well as extracts of Larrea divaricata, such as are contained for example and preferably in the raw material Capislow from Sederma, which contains lecithin vesicles with a hydroglycolized extract of Larrea divaricata.

The preferred compositions according to the invention contain at least one of the hair-growth inhibiting substances preferably in a quantity of 0.1% - 10% by weight, preferably 0.5% - 5% by weight and particularly preferred 1% - 4% by weight, related respectively to the weight of the raw material tel quel and the total weight of the combination according to the invention.

Preservatives

Preferably, the usual preservatives can also be added to the compositions according to the invention, in order to prevent the decomposition of the product through microbial growths. Numerous preservatives also necessarily have deodorizing characteristics, so that some substances belong to both groups. For cosmetics preferably suitable as preservatives are for example benzoic acid and its derivatives (e.g. propyl-, phenyl- and butylbenzoate, ammonium-, sodium-, potassium- and magnesiumbenzoate), propionic acid and its derivatives (e.g. ammonium-, sodium-, potassium- and magnesium propionate), salicylic acid and its derivatives (e.g. sodium-, potassium- and magnesiumsalicylate), 4-hydroxybenzoic acid and its esters and alkali-metal salts (e.g. methyl-, ethyl-, propyl-, isopropyl-, butyl-, isobutyl-, isodecyl-, phenyl-, phenoxyethyl- and benzylparabens, hexamidine parabens and di-parabens, sodium and potassium parabens, sodium and potassium methylparabens, potassium butylparabens, sodium and potassium propylparabens), aromatic alcohols and their salts (e.g. benzyl alcohol, phenethyl alcohol, phenol, potassium phenolate, phenoxyethanol, phenoxyisopropanol, o-phenylphenol), guajacol and its derivatives, chlorhexidine and its derivatives (e.g. chlorhexidindiacetate, -digluconate, and -dihydrochloride), hydantoin and its derivatives (e.g. DEDM- and DMDM-hydantoin, DEDM-Hydantoindilaurate), urea and urea derivatives (e.g. diazolidinyl urea, imidazolidi-nyl urea), ferulaic acid and its derivatives (e.g. ethylferulate), sorbinic acid and its derivatives (e.g. isopropylsorbate, TEA-sorbate, sodium-, potassium- and magnesiumsorbate), isothiazol and oxazol derivatives (e.g. methylisothiazolinone, methylchloroisothiazolinone, di-methyloxazolidine), quaternary ammonium compounds (e.g. Polyquaternium-42, Quaternium-8, Quaternium-14, Quaternium-15), carbamates (e.g. iodopropynylbutylcarbamate), formaldehyde and sodium formate, glutaraldehyde, glyoxal, hexamidine, dehydracetic acid, 2-bromo-2-nitropropane-1,3-diol, isopropylkresol, methyldibromoglutaronitrile, polyaminopropylbiguanide, sodium, hydroxymethylglycinate, sodium phenolsulfonate, triclocarban, triclosan, zinc pyrithione, as well as diverse peptide antibiotics (e.g. Nisine).

Preferred preservatives according to the invention are phenoxyethanol, the esters of 4-hydroxybenzoic acid, in particular methyl-, ethyl-, propyl-, isopropyl-, butyl- and isobutylparabens, as well as iodopropynylbutylcarbamate.

The quantity of the preservatives in the compositions preferred according to the invention is 0.001 % -10% by weight, preferably 0.01 % - 5% by weight and in particular 0.1% - 3% by weight, relating to the total weight of the composition.

In principle the subject of the present invention is to be extended to other cosmetic stick combinations, which do not represent deodorant or antiperspirant sticks. A content of deodorant or antiperspirant active substances is not obligatory in such sticks. Corresponding sticks for example can be mass-produced as lipsticks or concealer sticks and used through topical application on the skin.

A further subject of the present invention is a cosmetic, non-therapeutic process in order to minimize body odor, which is wherein a cosmetic deodorant and/or antiperspirant composition according to one of the Patent Claims 1 - 32 is to be applied on the skin, in particular on the skin of the armpits.

A further subject of the present invention is a process for the manufacture of a deodorant or antiperspirant stick according to one of the Claims 1 - 32, whereby the wax and oil components are heated up to 90° - 95° C and melted down along with the oil-in-water and the water-in-oil emulsifier(s), following which the water, which has similarly been heated to 90° - 95° C along with the water-soluble effective components is added, while being thoroughly stirred, further contents, optionally, are mixed in, the mixture is cooled to a suitable temperature for filling, is filled in suitable dispensers and solidified through static cooling (without being further stirred) at room temperature.

The following examples are meant to clarify the subject of the invention without limiting it to only these.

Table : Exemplary compositions according to the invention

| Example no. | 1 | 2 | 3 | 4 |
|---|---|---|---|---|
| Cutina ^{®} AGS | 2.4 | 2.4 | 2.4 | 2.4 |
| Cutina^{®} FS45 | 3.5 | 3.5 | 3.5 | 3.5 |
| Eumulgin^{®} B2 | 0.8 | 0.8 | 0.8 | 0.8 |
| Eumulgin^{®} B3 | 0.8 | 0.8 | 0.8 | 0.8 |
| Diisopropyladipate | 6 | 6 | 6 | 6 |
| Novata^{®} AB | 4 | 4 | 4 | 4 |
| Cutina^{®} CP | 5 | 5 | 5 | 5 |
| Cutina^{®} HR | 4 | 4 | 4 | 4 |
| Kesterwachs K62 | 5 | 5 | 5 | 5 |
| Locron^{®} L | 40 | 40 | 40 | 40 |
| Talkum Pharma G | 10 | 10 | 10 | 10 |
| Perfume | 1.2 | 1.2 | 1.2 | 1.2 |
| Tinogard Q | 0.025 | 0.05 | - | - |
| Tinogard AS | 0.025 | - | - | - |
| 1,2-propanediol | 10 | 10 | 7.3 | 10 |
| Ethanol (cosmetic, denatured) | 3 | 5 | 10 | 7.3 |
| Water, fully desalinated | 4.25 | 2.25 | - | - |
| Total | 100.0 | 100.0 | 100.0 | 100.0 |
| Penetration g-force [g] / TA15-cone 45° / 5 mm / 2,0 mm/s (average) | 518 | 458 | 347 | 350 |
| electrical resistance 27 mm [Kilo-Ohm, KΩ] | 38 | 39 | 32 | 40 |
| tactile solidity of the mass (sensory) | solid | solid | solid | solid |
| Solubility parameter oil [(cal/cm³)^{0,5}] | 8.46 | 8.46 | 8.46 | 8.46 |
| Solubility parameter W/O-emulsifier [(cal/cm³)^{0,5}] | 8.24 | 8.24 | 8.24 | 8.24 |
| Difference solubility parameter [(cal/cm³)^{0,5}] | 0.22 | 0.22 | 0.22 | 0.22 |

All quantities are provided in % by weight.

Table : Further exemplary compositions according to the invention

| Example no. | 5 | 6 | 7 | 8 | 9 | 10 |
|---|---|---|---|---|---|---|
| Lorol C 18 | - | - | - | 2 | - | - |
| Cutina^{®} EGMS | 2.4 | 2 | - | - | - | - |
| Cutina^{®} PES | - | - | 2 | - | - | - |
| Cutina^{®} MD | - | - | - | - | 2.4 | 2.4 |
| Cutina^{®} FS45 | 3.5 | 3.5 | 3.5 | 3.5 | 3.5 | 3.5 |
| Eumulgin^{®} B2 | 0.8 | 0.8 | 0.8 | 0.8 | 0.8 | 0.8 |
| Eumulgin^{®} B3 | 0.8 | 0.8 | 0.8 | 0.8 | 0.8 | 0.8 |
| Diisopropyladipate | 6 | 6 | 6 | 6 | 6 | 6 |
| Novata^{®} AB | 4 | 4 | 4 | 4 | 4 | 4 |
| Cutina^{®} CP | 5 | 5 | 5 | 5 | 5 | 5 |
| Cutina^{®} HR | 4 | 4 | 4 | 4 | 4 | 4 |
| Kesterwachs K62 | 5 | 5 | 5 | 5 | 5 | 5 |
| Locron^{®} L | 40 | 40 | 40 | 40 | 40 | 40 |
| Talkum Pharma G | 10 | 10 | 10 | 10 | 10 | 8 |
| Perfume | 1.2 | 1.2 | 1.2 | 1.2 | 1.2 | 1.2 |
| Tinogard Q | - | - | - | - | 0.05 | 0.05 |
| Trilon B liquid | - | - | - | 0.2 | - | - |
| 1,2-propanediol | 10 | 10 | 10 | 10 | 10 | 12 |
| Ethanol (cosmetic, denatured) | 5 | 5 | 5 | 5 | 5 | 5 |
| Water, fully desalinated | 2.3 | 2.7 | 2.7 | 2.5 | 2.25 | 2.25 |
| Total | 100.0 | 100.0 | 100.0 | 100.0 | 100.0 | 100.0 |
| tactile solidity of the mass (sensory) | solid | solid | solid | solid | solid | solid |
| Solubility parameter oil [(cal/cm³)^{0,5}] | 8.46 | 8.46 | 8.46 | 8.46 | 8.46 | 8.46 |
| Solubility parameter W/O emulsifier [(cal/cm³)^{0,5}] | 8.28 | 8.28 | 8.0-8.2 | 8.9 | 8.28 | 8.28 |
| Difference solubility parameter [(cal/cm³)^{0,5}] | 0.18 | 0.18 | 0.36 | -0.44 | 0.18 | 0.18 |

All quantities are provided in % by weight.

List of the raw materials used

| Component | INCI | Supplier/Manufacturer |
|---|---|---|
| Cutina^{®} AGS | Glycol Distearate | Cognis |
| Cutina^{®} EGMS | Glycol Stearate | Cognis |
| Cutina^{®} PES | Pentaerythrityl Distearate | Cognis |
| Cutina^{®} CP | Cetyl Palmitate | Cognis |
| Cutina^{®} FS45 | Palmitic Acid, Stearic Acid | Cognis |
| Cutina^{®} HR | Hydrogenated Castor Oil | Cognis |
| Eumulgin^{®} B2 | Ceteareth-20 | Cognis |
| Eumulgin^{®} B3 | Ceteareth-30 | Cognis |
| Kesterwachs K62 | Cetearyl Behenate | Koster Keunen |
| Locron L (50 % ACH solution) | Aluminum Chlorohydrate | Clariant |
| Lorol C 18 | Stearyl Alcohol | Cognis |
| Novata^{®} AB | Cocoglycerides | Cognis |
| Tinogard AS | Bumetrizole | Ciba |
| Tinogard Q | Tris(tetramethylhydroxypiperidinol) citrate | Ciba |
| Trilon B liquid | Water, TETRASODIUM EDTA (39 - 41 % by weight active matter) | BASF |

## Claims

1. Deodorant or antiperspirant stick in the form of an oil-in-water dispersion/emulsion, containing
a) at least one lipid or wax component with a melting point > 50°C, which is not to be apportioned to the components b) or c),
b) at least one non-ionic oil-in-water emulsifier with an HLB value of more than 7 within an oil-in-water emulsifier mixture with an average HLB value in the range of 10-19,
c) at least one non-ionic water-in-oil emulsifier with an HLB value of more than 1.0 and less than/equal to 7.0, which can form liquid crystalline structures with water alone or with water in the presence of a hydrophilic emulsifier, as consistency regulator and/or water binder,
d) at least one oil which is in a liquid state at 20° C and is not a fragrance component or essential oil, in which the maximum deviation between the (average) solubility parameter of all the constituent oils d) and the (average) solubility parameter of the water-in-oil emulsifier or emulsifiers is -0.7 (cal/cm³)^{0.5} or +0.7 (cal/cm³)^{0.5}, respectively, in the presence of linear saturated fatty alcohols with a chain length of at least 8 carbon atoms and -0.4 (cal/cm³)^{0.5} or +0.7 (cal/cm³)^{0.5}, respectively, in the presence of water-in-oil emulsifiers other than linear saturated fatty alcohols with a chain length of at least 8 carbon atoms, linear saturated fatty alcohols with a chain length of at least 8 carbon atoms being absent and the share of silicon oils and/or hydrocarbons being not more than 20% in relation to the total weight of oils, which are liquid at 20°C,
e) at least one water-soluble polyhydric C₂ - C₉-alkanol with 2-6 hydroxyl groups and/or at least one water-soluble polyethylene glycol with 3-20 ethylene oxide units,
f) 5% up to less than 50 % by weight of water, relative to the total composition,
g) 1% to 15% by weight of ethanol and/or isopropanol, relative to the overall composition,
h) at least one deodorant or antiperspirant active substance.

2. Deodorant or antiperspirant stick according to Claim 1, wherein the lipid or wax component a) is chosen from esters of a saturated monohydric C₁₆-C₆₀-alkanol and a saturated C₈-C₃₆-monocarboxylic acid, in particular cetylbehenate, stearylbehenate and C₂₀-C₄₀-alkylstearate, glycerine tri-esters of saturated linear C₁₂-C₃₀-carboxylic acids, which can be hydroxylated, candelilla wax, carnauba wax, beeswax, saturated linear C₁₄-C₃₆-carboxylic acids, as well as mixtures of the substances named above.

3. Deodorant or antiperspirant stick according to one of the preceding claims, wherein the lipid or wax component a) is contained in all in quantities of 4% - 20% by weight, preferably 8% - 15% by weight, relative to the total composition.

4. Deodorant or antiperspirant stick according to one of the preceding claims, wherein the ester(s) of a saturated, monohydric C₁₆-C₆₀-alkanol and a saturated C₈-C₃₆-monocarboxylic acid is/are contained in quantities of 2%
- 10% by weight, preferably 2% - 6% by weight, relative to the total composition.

5. Deodorant or antiperspirant stick according to one of the preceding claims, wherein the non-ionic oil-in-water emulsifier b) with an HLB value of more than 7 is chosen from
- ethoxylated C₈-C₂₄-alkanols with an average of 10-100 mol ethylene oxide per mol,
- ethoxylated C₈-C₂₄-carboxylic acids with an average of 10 - 100 mol ethylene oxide per mol,
- silicone-copolyols with ethylene oxide units or with ethylene oxide-and propylene oxide units,
- alkyl mono- and -oligoglycosides with 8 to 22 carbon atoms in the alkyl residue and their ethoxylated analogs,
- ethoxylated sterols,
- partial esters of polyglycerines with 2 to 10 glycerine units and esterified with 1 to 4 saturated or unsaturated, linear or branched C₈ - C₂₂-fatty acid residues, as long as they show an HLB value of more than 7,
- as well as mixtures of the substances named above.

6. Deodorant or antiperspirant stick according to one of the preceding claims, wherein the oil-in-water emulsifier b) is contained in a total quantity of 0.5%
- 10% by weight, preferably 1% - 4% by weight, relating to the total composition.

7. Deodorant or antiperspirant stick according to one of the preceding claims, wherein at least one further non-ionic water-in-oil emulsifier is contained, which is chosen from
- ethylene glycol mono- and -di-esters of linear, saturated and unsaturated C₁₂ - C₃₀-carboxylic acids, which can be hydroxylated,
- pentaerythrityl mono-, -di-, tri- and tetraesters of linear, saturated and unsaturated C₁₂ - C₃₀-carboxylic acids, which can be hydroxylated,
- linear, saturated C₁₂ - C₃₀-alkanols,
- glyceryl mono- and di-esters of linear, saturated and unsaturated C₁₂ - C₃₀-carboxylic acids, which can be hydroxylated,
- propylene glycol mono- and -di-esters of linear, saturated and unsaturated C₁₂ - C₃₀-carboxylic acids, which can be hydroxylated,
- sorbitane mono-, -di- and -tri-esters of linear, saturated and unsaturated C₁₂ - C₃₀-carboxylic acids, which can be hydroxylated,
- methyl glucose mono- and -di-esters of linear, saturated and unsaturated C₁₂ - C₃₀-carboxylic acids, which can be hydroxylated,
- sterols,
- alkanols and carboxylic acids with 8 - 24 C atoms respectively, in particular with 16-22 C atoms, in the alkyl or acyl group and 1-4 ethylene oxide units per molecule, which show an HLB value of more than 1.0 and less than/equal to 7.0,
- glyceryl mono-ethers of saturated and/or unsaturated, branched and/or unbranched alcohols of a chain length of 8 - 30, in particular 12-18 carbon atoms,
- partial esters of polyglycerines with n = 2 to 10 glycerine units and esterified with 1 to 5 saturated or unsaturated, linear or branched, optionally hydroxylated C₈ - C₃₀-fatty acid residues, as long as they show an HLB value of less than/equal to 7,
- as well as mixtures of the substances named above.

8. Deodorant or antiperspirant stick according to one of the preceding claims, wherein the water-in-oil emulsifier c) is chosen from ethylene glycol monostearate, ethylene glycol distearate, pentaerythrityl monostearate, pentaerythrityl di-stearate, pentaerythrityl tristearate and pentaerythrityl tetrastearate and mixtures thereof.

9. Deodorant or antiperspirant stick according to one of the preceding claims, wherein the water-in-oil emulsifier(s) c) is/are contained in a total quantity of 0.1% - 15% by weight, preferably 0.5% - 8.0% by weight, and particularly preferred 1% - 4% by weight, relative to the total composition.

10. Deodorant or antiperspirant stick according to one of the preceding claims, wherein the oil d), liquid at 20° C, is chosen from
- di-carboxylic acid esters of linear or branched C₂-C₁₀-alkanols,
- branched saturated or unsaturated fatty alcohols with 6-30 carbon atoms,
- triglycerides of linear or branched, saturated or unsaturated, optionally hydroxylated C₈₋₃₀-fatty acids,
- esters of branched saturated or unsaturated fatty alcohols with 2 - 30 carbon atoms with linear or branched, saturated or unsaturated fatty acids with 2-30 carbon atoms, which can be hydroxylated,
- addition products of 1 to 5 propylene oxide units to monohydric or polyhydric C₈₋₂₂-alkanols,
- addition products of at least 6 propylene oxide units to monohydric or polyhydric C₃₋₂₂-alkanols,
- C₈-C₂₂-fatty alcohol esters of monovalent or polyvalent C₂-C₇-hydroxycarboxylic acids,
- symmetric, asymmetric or cyclic esters of carbonic acid with fatty alcohols,
- the esters of dimers of unsaturated C₁₂-C₂₂-fatty acids (dimeric fatty acids) with monohydric linear, branched or cyclic C₂-C₁₈-alkanols or with polyhydric linear or branched C₂-C₆-alkanols,
- as well as mixtures of the substances named above.

11. Deodorant or antiperspirant stick according to one of the preceding claims, wherein the oil(s) d), liquid at 20°C, is/are contained in a total quantity of 3% - 20% by weight, preferably 5% - 14% by weight, relating to the total weight of the composition.

12. Deodorant or antiperspirant stick according to one of the preceding claims, wherein the oils d), liquid at 20° C, contain a maximum of 20% by weight of oil(s) related to the total weight of oils liquid at 20° C, the solubility parameter of which differs by more than -0.4 resp. -0.7 (cal/cm³)^{0,5} or by more than +0.7 (cal/cm³)^{0,5} from the (average) solubility parameter of the water-in-oil emulsifier(s) c).

13. Deodorant or antiperspirant stick according to one of the preceding claims, wherein no oils liquid at 20° C are contained, the solubility parameter of which differs by more than ± 1.0 (cal/cm³)^{0,5} from the (average) solubility parameter of the water-in-oil emulsifier(s) c).

14. Deodorant or antiperspirant stick according to one of the preceding claims, wherein the water-soluble polyhydric C₂ - C₉-alkanol e) with 2-6 hydroxyl groups is chosen from 1,2-propylene glycol, 2-methyl-1,3-propanediol, glycerine, butylene glycols such as 1,2-butylene glycol, 1,3-butylene glycol and 1,4-butylene glycol, pentylene glycols such as 1,2-pentanediol and 1,5-pentanediol, hexanediols such as 1,6-hexanediol, hexanetriols such as 1,2,6-hexanetriol, 1,2-octanediol, 1,8-octanediol, dipropylene glycol, tripropylene glycol, diglycerine, triglycerine, erythritol, sorbitol as well as mixtures of the substances named above. -

15. Deodorant or antiperspirant stick according to one of the preceding claims, wherein the water-soluble polyhydric C₂ - C₉-alkanol with 2-6 hydroxyl groups and/or the water-soluble polyethylene glycol with 3 - 20 ethylene oxide units is contained in all in quantities of 3% - 25% by weight, preferably 8% - 18% by weight.

16. Deodorant or antiperspirant stick according to one of the preceding claims, wherein further at least one lipid or wax component with a melting point in the range of 25° - < 50° C is contained, chosen from coconut fatty acid glycerine mono-, di- and tri- esters, Butyrospermum parkii (Shea Butter) and esters of saturated, monohydric C₈-C₁₈-alcohols with saturated C₁₂-C₁₈-monocarboxylic acids, as well as mixtures of these substances.

17. Deodorant or antiperspirant stick according to Claim 16, wherein the lipid or wax component with a melting point in the range of 25° - < 50° C is contained in quantities of 0.01% to 20% by weight, preferably 3% - 20% by weight, particularly preferred 5% - 18% by weight and extraordinarily preferred 6% - 15% by weight, relative to the total composition.

18. Deodorant or antiperspirant stick according to one of the preceding claims, wherein the total content of ethanol and/or isopropanol is 2% to 10% by weight, particularly preferred 3% to 8% by weight, exceptionally preferred 5% to 7% by weight, relative respectively to the total composition.

19. Deodorant or antiperspirant stick according to one of the preceding claims, wherein the deodorant active substance is chosen from
- arylsulfatase inhibitors, β-glucuronidase inhibitors, aminoacylase inhibitors, esterase inhibitors, lipase inhibitors and lipoxigenase inhibitors,
- α-monoalkyl glycerine ethers with one-branched or linear saturated or unsaturated, optionally hydroxylated C₆ - C₂₂-alkyl residue, in particular α-(2-ethylhexyl)glycerine ether,
- phenoxyethanol,
- perfumed germ inhibiting oils,
- Deosafe^{®} perfumed oils,
- components with pre-biotic action,
- trialkylcitric acid esters, in particular triethylcitrate,
- substances, which reduce the number of the odor causing skin bacteria of the group of staphylococci, coryne bacteria xerosis, coryne bacterium, anaerococci and micrococci, resp. which inhibit their growth,
- zinc compounds, in particular zinc phenolsulfonate and zinc ricinoleate,
- organo-halogen compounds, in particular triclosan, chlorhexidine, chlorhexidine gluconate and benzalkonium halogenides,
- quaternary ammonium compounds, in particular cetylpyridinium chloride,
- odor absorbers, in particular silicates and zeolithes,
- sodium bicarbonate,
- lantibiotics,
- as well as mixtures of the substances named above, and/or the antiperspirant active substance is chosen from the water-soluble, astringent, inorganic and organic salts of aluminum, zirconium and zinc, resp. any mixtures of these salts.

20. Deodorant or antiperspirant stick according to one of the preceding claims, wherein the deodorant active substance, is contained in a total quantity of 0.1% - 10% by weight, preferably 0.2% - 7% by weight, particularly preferred 0.3% - 5% by weight and exceptionally preferred 0.4%-1.0% by weight, relating to 0.1% - 10 % by weight, and/or the antiperspirant active substance is contained in a total quantity of 3% - 25% by weight, preferably 5% - 22% by weight and in particular 10% - 20% by weight, relating respectively to the total weight of the active substance in the total composition.

21. Deodorant or antiperspirant stick according to one of the preceding claims, wherein further at least one solid, water-insoluble particulate filler is contained.

22. Deodorant or antiperspirant stick according to one of the preceding claims, wherein the solid, water-insoluble particulate filler is chosen from - optionally modified - starches and starch derivatives, which are, if desired, pre-gelatinized, cellulose and cellulose derivatives, silicon dioxide, silicic acids, spherical polyalkylsesquisiloxan particles, silicic gels (silica), talcum, kaolin, clays, e.g. bentonites, magnesium aluminum silicates, bornitride, lactoglobuline derivatives, glass powder, polymer powders as well as mixtures of the substances named above.

23. Deodorant or antiperspirant stick according to one of the preceding claims, wherein the solid, water-insoluble particulate filler is contained in a total quantity of 0.01% to 30% by weight, preferably 5% - 20% by weight, particularly preferred 8% - 15% by weight, relative respectively to the total composition.

24. Deodorant or antiperspirant stick according to one of the preceding claims, wherein 10% to less than 30% by weight of water, relative to the total composition, are contained.

25. Deodorant or antiperspirant stick according to one of the preceding claims, wherein the total content of nonionic and ionic emulsifiers and/or surfactants is with an HLB value of over 8 with a maximum of 20% by weight, preferably a maximum of 15% by weight, particularly preferred a maximum of 10% by weight, particularly preferred a maximum of 7% by weight, further particularly preferred a maximum of 4% by weight and exceptionally preferred a maximum of 3% by weight, relative respectively to the total composition of the invention.

26. Stick composition according to one of the preceding claims, **characterized by** a penetration force value in the range of 150 - 800 gram force (g-force), 250 - 600 gram force (g-force), particularly preferred 300 - 520 gram force (g-force), extraordinarily preferred 340 - 500 gram force (g-force), at a penetration depth of 5.000 mm.

27. Stick composition according to one of the preceding claims, **characterized by** an electrical resistance of a maximum of 300 kΩ, preferably a maximum of 100 kΩ and particularly preferred a maximum of 80 kΩ.

28. Deodorant or antiperspirant stick according to one of the preceding claims, wherein further at least one scent component is contained.

29. Deodorant or antiperspirant stick according to one of the preceding claims, wherein at least one radical capturing substance is contained.

30. Deodorant or antiperspirant stick according to one of the preceding claims, wherein at least one UV filter is contained.

31. Deodorant or antiperspirant stick according to one of the preceding claims, wherein at least one complexing substance is contained.

32. Deodorant or antiperspirant stick according to one of the preceding claims, wherein at least one hair-growth inhibiting substance is contained.

33. Cosmetic, non-therapeutic process for the reduction of body odor, wherein a cosmetic deodorant and/or antiperspirant composition according to one of the Claims 1 - 32 is applied on the skin, in particular on the skin of the armpits.

34. Process for the manufacture of a deodorant or antiperspirant stick according to one of the Claims 1 - 32, whereby the wax and oil components along with the oil-in-water- and the water-in-oil emulsifier(s) is/are heated to 90° - 95°C and melted, following which the water also heated to 90° - 95°C is added along with the water-soluble active substances and ingredients, while being stirred vigorously; further ingredients are optionally mixed in, the mixture is cooled to a suitable filling temperature, filled into suitable dispensers and solidified at room temperature through static cooling (without further stirring).

## Patentansprüche

1. Deodorant- oder Antitranspirant-Stift in Form einer Öl-in-Wasser-Dispersion/Emulsion, enthaltend
a) mindestens eine Lipid- oder Wachskomponente mit einem Schmelzpunkt > 50°C, die nicht den Komponenten b) oder c) zuzurechnen ist,
b) mindestens einen nichtionischen Öl-in-Wasser-Emulgator mit einem HLB-Wert von mehr als 7 innerhalb eines Öl-in-Wasser-Emulgatorgemisches mit einem mittleren HLB-Wert im Bereich von 10-19,
c) mindestens einen nichtionischen Wasser-in-Öl-Emulgator mit einem HLB-Wert größer 1,0 und kleiner/gleich 7,0, der mit Wasser allein oder mit Wasser in Gegenwart eines hydrophilen Emulgators flüssigkristalline Strukturen ausbilden kann, als Konsistenzgeber und/oder Wasserbinder,
d) mindestens ein bei 20 °C flüssiges Öl, das keine Duftstoffkomponente und kein etherisches Öl darstellt, wobei der (mittlere) Löslichkeitsparameter der Gesamtheit der enthaltenen Öle in Gegenwart von linearen gesättigten Fettalkoholen mit einer Kettenlänge von mindestens 8 Kohlenstoffatomen um maximal -0,7 (cal/cm³)^{0,5} bzw. maximal +0,7 (cal/cm³)^{0,5}, und in Gegenwart von Wasser-in-Öl-Emulgatoren, die von linearen gesättigten Fettalkoholen mit einer Kettenlänge von mindestens 8 Kohlenstoffatomen verschieden sind, in Abwesenheit von linearen gesättigten Fettalkoholen mit einer Kettenlänge von mindestens 8 Kohlenstoffatomen um maximal -0,4 (cal/cm³)^{0,5} bzw. maximal +0,7 (cal/cm³)^{0,5} vom (mittleren) Löslichkeitsparameter des Wasser-in-Öl-Emulgators/der Wasser-in-Öl-Emulgatoren abweicht,
wobei der Anteil an Siliconölen und/oder Kohlenwasserstoffen nicht größer ist als 20 %, bezogen auf das Gesamtgewicht an bei 20°C flüssigen Ölen,
e) mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten,
f) 5 bis weniger als 50 Gew.-% Wasser, bezogen auf die Gesamtzusammensetzung,
g) 1 bis 15 Gew.-% Ethanol und/oder Isopropanol, bezogen auf die Gesamtzusammensetzung,
h) mindestens einen Deodorant- oder Antitranspirant-Wirkstoff.

2. Deodorant- oder Antitranspirant-Stift gemäß Anspruch 1, **dadurch gekennzeichnet, dass** die Lipid- oder Wachskomponente a) ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen.

3. Deodorant- oder Antitranspirant-Stift gemäß einem der vorstehenden Ansprüche, **dadurch gekennzeichnet, dass** die Lipid- oder Wachskomponente a) insgesamt in Mengen von 4-20 Gew.-%, bevorzugt 8-15 Gew.-%, bezogen auf die Gesamtzusammensetzung, enthalten ist.

4. Deodorant- oder Antitranspirant-Stift gemäß einem der vorstehenden Ansprüche, **dadurch gekennzeichnet, dass** der/die Ester aus einem gesättigten, einwertigen C₁₆-C₆₀-Alkanol und einer gesättigten C₈-C₃₆-Monocarbonsäure in Mengen von 2-10 Gew.-%, bevorzugt 2-6 Gew.-%, bezogen auf die Gesamtzusammensetzung, enthalten ist/sind.

5. Deodorant- oder Antitranspirant-Stift gemäß einem der vorstehenden Ansprüche, **dadurch gekennzeichnet, dass** der nichtionische Öl-in-Wasser-Emulgator b) mit einem HLB-Wert von mehr als 7 ausgewählt ist aus
- ethoxylierten C₈-C₂₄-Alkanolen mit durchschnittlich 10-100 Mol Ethylenoxid pro Mol,
- ethoxylierten C₈-C₂₄-Carbonsäuren mit durchschnittlich 10-100 Mol Ethylenoxid pro Mol,
- Silicon-Copolyolen mit Ethylenoxid-Einheiten oder mit Ethylenoxid- und Propylenoxid-Einheiten,
- Alkylmono- und -oligoglycosiden mit 8 bis 22 Kohlenstoffatomen im Alkylrest und deren ethoxylierten Analoga,
- ethoxylierten Sterinen,
- Partialestern von Polyglycerinen mit 2 bis 10 Glycerineinheiten und mit 1 bis 4 gesättigten oder ungesättigten, linearen oder verzweigten C₈ - C₃₀-Fett-säureresten verestert, sofern sie einen HLB-Wert von mehr als 7 aufweisen,
- sowie Mischungen der vorgenannten Substanzen.

6. Deodorant- oder Antitranspirant-Stift gemäß einem der vorstehenden Ansprüche, **dadurch gekennzeichnet, dass** der Öl-in-Wasser-Emulgator b) in einer Gesamtmenge von 0,5-10 Gew.-%, bevorzugt 1-4 Gew.-%, bezogen auf die Gesamtzusammensetzung, enthalten ist.

7. Deodorant- oder Antitranspirant-Stift gemäß einem der vorstehenden Ansprüche, **dadurch gekennzeichnet, dass** der nichtionische Wasser-in-Öl-Emulgator ausgewählt ist aus
- Ethylenglycolmono- und -diestern von linearen, gesättigten und ungesättigten C₁₂ - C₃₀-Carbonsäuren, die hydroxyliert sein können,
- Pentaerythritylmono-, -di-, -tri- und -tetraestern von linearen, gesättigten und ungesättigten C₁₂ - C₃₀-Carbonsäuren, die hydroxyliert sein können,
- linearen, gesättigten C₁₂ - C₃₀-Alkanolen,
- Glycerinmono- und diestern von linearen, gesättigten und ungesättigten C₁₂ - C₃₀-Carbonsäuren, die hydroxyliert sein können,
- Propylenglycolmono- und -diestern von linearen, gesättigten und ungesättigten C₁₂ - C₃₀-Carbonsäuren, die hydroxyliert sein können,
- Sorbitanmono-, -di- und -triestern von linearen, gesättigten und ungesättigten C₁₂ - C₃₀-Carbon-säuren, die hydroxyliert sein können,
- Methylglucosemono- und -diestern von linearen, gesättigten und ungesättigten C₁₂ - C₃₀-Carbonsäuren, die hydroxyliert sein können,
- Sterinen,
- Alkanolen und Carbonsäuren mit jeweils 8-24 C-Atomen, insbesondere mit 16-22 C-Atomen, in der Alkyl- oder Acylgruppe und 1-4 Ethylenoxid-Einheiten pro Molekül, die einen HLB-Wert größer 1,0 und kleiner/gleich 7,0 aufweisen,
- Glycerinmonoethern gesättigter und/oder ungesättigter, verzweigter und/oder unverzweigter Alkohole einer Kettenlänge von 8 - 30, insbesondere 12-18 Kohlenstoffatomen,
- Partialestern von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich 7 aufweisen,
- sowie Mischungen der vorgenannten Substanzen.

8. Deodorant- oder Antitranspirant-Stift gemäß einem der vorstehenden Ansprüche, **dadurch gekennzeichnet, dass** der Wasser-in-Öl-Emulgator c) ausgewählt ist aus Ethylenglycolmonostearat, Ethylenglycoldistearat, Pentaerythritylmonostearat, Pentaerythrityldistearat, Pentaerythrityltristearat und Pentaerythrityltetrastearat sowie Mischungen hiervon.

9. Deodorant- oder Antitranspirant-Stift gemäß einem der vorstehenden Ansprüche, **dadurch gekennzeichnet, dass** der Wasser-in-Öl-Emulgator c) in einer Gesamtmenge von 0,1 - 15 Gew.-%, bevorzugt 0,5 - 8,0 Gew.-%, und besonders bevorzugt 1-4 Gew.-%, bezogen auf die Gesamtzusammensetzung, enthalten ist.

10. Deodorant- oder Antitranspirant-Stift gemäß einem der vorstehenden Ansprüche, **dadurch gekennzeichnet, dass** das bei 20°C flüssige Öl d) ausgewählt ist aus
- Dicarbonsäureestern von linearen oder verzweigten C₂-C₁₀-Alkanolen,
- verzweigten gesättigten oder ungesättigten Fettalkoholen mit 6-30 Kohlenstoffatomen,
- Triglyceriden von linearen oder verzweigten, gesättigten oder ungesättigten, gegebenenfalls hydroxylierten C₈₋₃₀-Fettsäuren,
- Estern von verzweigten gesättigten oder ungesättigten Fettalkoholen mit 2 - 30 Kohlenstoffatomen mit linearen oder verzweigten gesättigten oder ungesättigten Fettsäuren mit 2-30 Kohlenstoffatomen, die hydroxyliert sein können,
- Anlagerungsprodukten von 1 bis 5 Propylenoxid-Einheiten an ein- oder mehrwertige C₈₋₂₂-Alkanole,
- Anlagerungsprodukten von mindestens 6 Ethylenoxid und/oder Propylenoxid-Einheiten an ein- oder mehrwertige C₃₋₂₂-Alkanole,
- C₈-C₂₂-Fettalkoholestern einwertiger oder mehrwertiger C₂-C₇-Hydroxycarbonsäuren,
- symmetrischen, unsymmetrischen oder cyclischen Estern der Kohlensäure mit Fettalkoholen,
- den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen,
- sowie Mischungen der vorgenannten Substanzen.

11. Deodorant- oder Antitranspirant-Stift gemäß einem der vorstehenden Ansprüche, **dadurch gekennzeichnet, dass** das/die bei 20 ° C flüssige/n Öl/e d) in einer Gesamtmenge von 3-20 Gew.-%, bevorzugt 5-14 Gew.-%, bezogen auf das Gesamtgewicht der Zusammensetzung, enthalten ist/sind.

12. Deodorant- oder Antitranspirant-Stift gemäß einem der vorstehenden Ansprüche, **dadurch gekennzeichnet, dass** die bei 20°C flüssigen Öle d) maximal 20 Gew.-% Öl/e, bezogen auf das Gesamtgewicht an bei 20°C flüssigen Ölen, enthalten, deren Löslichkeitsparameter um mehr als -0,4 bzw. -0,7 (cal/cm³)^{0,5} oder um mehr als + 0,7 (cal/cm³)^{0,5} vom (mittleren) Löslichkeitsparameter des Wasser-in-Öl-Emulgators/ der Wasser-in-Öl-Emulgatoren c) abweicht.

13. Deodorant- oder Antitranspirant-Stift gemäß einem der vorstehenden Ansprüche, **dadurch gekennzeichnet, dass** keine bei 20°C flüssigen Öle enthalten sind, deren Löslichkeitsparameter um mehr als ± 1,0 (cal/cm³)^{0,5} vom (mittleren) Löslichkeitsparameter des Wasser-in-Öl-Emulgators/der Wasser-in-Öl-Emulgatoren c) abweicht.

14. Deodorant- oder Antitranspirant-Stift gemäß einem der vorstehenden Ansprüche, **dadurch gekennzeichnet, dass** das wasserlösliche mehrwertige C₂ - C₉-Alkanol e) mit 2-6 Hydroxylgruppen ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen.

15. Deodorant- oder Antitranspirant-Stift gemäß einem der vorstehenden Ansprüche, **dadurch gekennzeichnet, dass** das wasserlösliche mehrwertige C₂ - C₉-Alkanol mit 2 - 6 Hydroxylgruppen und/oder das wasserlösliche Polyethylenglykol mit 3-20 Ethylenoxid-Einheiten insgesamt in Mengen von 3-25 Gew.-%, bevorzugt 8-18 Gew.-%, bezogen auf die Gesamtzusammensetzung, enthalten ist.

16. Deodorant- oder Antitranspirant-Stift gemäß einem der vorstehenden Ansprüche, **dadurch gekennzeichnet, dass** weiterhin mindestens eine Lipid- oder Wachskomponente mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist.

17. Deodorant- oder Antitranspirant-Stift gemäß Anspruch 16, **dadurch gekennzeichnet, dass** die Lipid- oder Wachskomponente mit einem Schmelzpunkt im Bereich von 25 - < 50°C in Mengen von 0,01 bis 20 Gew.-%, bevorzugt 3-20 Gew.-%, besonders bevorzugt 5-18 Gew.-% und außerordentlich bevorzugt 6-15 Gew.-%, bezogen auf die Gesamtzusammensetzung, enthalten ist.

18. Deodorant- oder Antitranspirant-Stift gemäß einem der vorstehenden Ansprüche, **dadurch gekennzeichnet, dass** der Ethanol- und/oder Isopropanolgehalt insgesamt 2 - 10 Gew.-%, bevorzugt 3-8 Gew.-% und besonders bevorzugt 5-7 Gew.-%, jeweils bezogen auf die Gesamtzusammensetzung, beträgt.

19. Deodorant- oder Antitranspirant-Stift gemäß einem der vorstehenden Ansprüche, **dadurch gekennzeichnet, dass** der Deodorant-Wirkstoff ausgewählt ist aus
- Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren,
- α-Monoalkylglycerinethern mit einem verzweigten oder linearen gesättigten oder ungesättigten, gegebenenfalls hydroxylierten C₆ - C₂₂-Alkylrest, insbesondere α-(2-Ethylhexyl)glycerinether,
- Phenoxyethanol,
- keimhemmend wirkenden Parfümölen,
- Deosafe-Parfümölen,
- präbiotisch wirksamen Komponenten,
- Trialkylcitronensäureestern, insbesondere Triethylcitrat,
- Wirkstoffen, die die Zahl der an der Geruchsbildung beteiligten Hautkeime aus der Gruppe der Staphylokokken, Corynebakterium xerosis, Corynebakterien, Anaerokokken und Mikrokokken reduzieren bzw. deren Wachstum hemmen,
- Zinkverbindungen, insbesondere Zinkphenolsulfonat und Zinkricinoleat,
- Organohalogenverbindungen, insbesondere Triclosan, Chlorhexidin, Chlorhexidingluconat und Benzalkoniumhalogeniden,
- quartären Ammoniumverbindungen, insbesondere Cetylpyridiniumchlorid,
- Geruchsabsorbern, insbesondere Silikaten und Zeolithen,
- Natriumbicarbonat,
- Lantibiotika,
- sowie Mischungen der vorgenannten Substanzen,
und/oder der Antitranspirant-Wirkstoff ausgewählt ist aus den wasserlöslichen adstringierenden anorganischen und organischen Salzen des Aluminiums, Zirkoniums und Zinks bzw. beliebigen Mischungen dieser Salze.

20. Deodorant- oder Antitranspirant-Stift gemäß einem der vorstehenden Ansprüche, **dadurch gekennzeichnet, dass** der Deodorant-Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%, bezogen auf von 0,1 - 10 Gew.-%, und/oder der Antitranspirant-Wirkstoff in einer Gesamtmenge von 3-25 Gew.-%, vorzugsweise 5-22 Gew.-% und insbesondere 10-20 Gew.-%, jeweils bezogen auf das Gesamtgewicht der Aktivsubstanz in der Gesamtzusammensetzung, enthalten ist.

21. Deodorant- oder Antitranspirant-Stift gemäß einem der vorstehenden Ansprüche, **dadurch gekennzeichnet, dass** weiterhin mindestens ein fester, wasserunlöslicher teilchenförmiger Füllstoff enthalten ist.

22. Deodorant- oder Antitranspirant-Stift gemäß einem der vorstehenden Ansprüche, **dadurch gekennzeichnet, dass** der feste, wasserunlösliche teilchenförmige Füllstoff ausgewählt ist aus gegebenenfalls modifizierten Stärken und Stärkederivaten, die gewünschtenfalls vorverkleistert sind, Cellulose und Cellulosederivaten, Siliciumdioxid, Kieselsäuren, sphärischen Polyalkylsesquioxan-Partikeln, Kieselgelen (Silica), Talkum, Kaolin, Tonen, z. B. Bentoniten, Magnesiumaluminiumsilikaten, Bornitrid, Lactoglobulinderivaten, Glaspulver, Polymerpulvern sowie Mischungen der vorgenannten Substanzen.

23. Deodorant- oder Antitranspirant-Stift gemäß einem der vorstehenden Ansprüche, **dadurch gekennzeichnet, dass** der feste, wasserunlösliche, teilchenförmige Füllstoff in einer Gesamtmenge von 0,01 bis 30 Gew.-%, bevorzugt 5-20 Gew.-%, besonders bevorzugt 8-15 Gew.-%, jeweils bezogen auf die Gesamtzusammensetzung, enthalten ist.

24. Deodorant- oder Antitranspirant-Stift gemäß einem der vorstehenden Ansprüche, **dadurch gekennzeichnet, dass** 10 bis weniger als 30 Gew.-% Wasser, bezogen auf die Gesamtzusammensetzung, enthalten sind.

25. Deodorant- oder Antitranspirant-Stift gemäß einem der vorstehenden Ansprüche, **dadurch gekennzeichnet, dass** der Gesamtgehalt an nichtionischen und ionischen Emulgatoren und/oder Tensiden mit einem HLB-Wert über 8 maximal 20 Gew.-%, bevorzugt maximal 15 Gew.-%, besonders bevorzugt maximal 10 Gew.-%, weiter besonders bevorzugt maximal 7 Gew.-%, weiter besonders bevorzugt maximal 4 Gew.-% und außerordentlich bevorzugt maximal 3 Gew.-%, beträgt, jeweils bezogen auf die gesamte erfindungsgemäße Zusammensetzung.

26. Stiftzusammensetzung gemäß einem der vorstehenden Ansprüche, **gekennzeichnet durch** einen Penetrationskraftwert im Bereich von 150 - 800 Gramm-Kraft (g-force), 250 - 600 Gramm-Kraft (g-force), besonders bevorzugt 300 - 520 Gramm-Kraft (g-force) und außerordentlich bevorzugt 340 - 500 Gramm-Kraft (g-force), bei einer Penetrationstiefe von 5,000 mm.

27. Stiftzusammensetzung gemäß einem der vorstehenden Ansprüche, **gekennzeichnet durch** einen elektrischen Widerstand von maximal 300 kΩ, bevorzugt maximal 100 kΩ, besonders bevorzugt maximal 80 kΩ.

28. Deodorant- oder Antitranspirant-Stift gemäß einem der vorstehenden Ansprüche, **dadurch gekennzeichnet, dass** weiterhin mindestens eine Duftstoffkomponente enthalten ist.

29. Deodorant- oder Antitranspirant-Stift gemäß einem der vorstehenden Ansprüche, **dadurch gekennzeichnet, dass** mindestens eine Radikalfängersubstanz enthalten ist.

30. Deodorant- oder Antitranspirant-Stift gemäß einem der vorstehenden Ansprüche, **dadurch gekennzeichnet, dass** mindestens ein UV-Filter enthalten ist.

31. Deodorant- oder Antitranspirant-Stift gemäß einem der vorstehenden Ansprüche, **dadurch gekennzeichnet, dass** mindestens eine komplexbildende Substanz enthalten ist.

32. Deodorant- oder Antitranspirant-Stift gemäß einem der vorstehenden Ansprüche, **dadurch gekennzeichnet, dass** mindestens eine den Haarwuchs inhibierende Substanz enthalten ist.

33. Kosmetisches, nicht-therapeutisches Verfahren zur Verringerung von Körpergeruch, **dadurch gekennzeichnet, dass** eine kosmetische Deodorant- und/oder Antitranspirant-Zusammensetzung nach einem der Ansprüche 1 - 32 auf die Haut, insbesondere auf die Haut der Achselhöhlen, aufgetragen wird.

34. Verfahren zur Herstellung eines Deodorant- oder Antitranspirant-Stiftes gemäß einem der Ansprüche 1 - 32, wobei die Wachs- und Ölkomponenten zusammen mit dem/den Öl-in-Wasser- und dem/den Wasser-in-Öl-Emulgator/en auf 90 - 95°C erhitzt und aufgeschmolzen werden, danach das ebenfalls auf 90 - 95°C erhitzte Wasser mit den wasserlöslichen Wirk- und Inhaltsstoffen unter kräftigem Rühren zugegeben wird, gegebenenfalls weitere Inhaltsstoffe beigemischt werden, die Mischung bis auf eine geeignete Abfülltemperatur abgekühlt, in geeignete Spenderformen abgefüllt und durch statisches Abkühlen (ohne weiteres Rühren) auf Raumtemperatur verfestigt wird.

## Revendications

1. Bâtonnet déodorant ou anti-transpiration sous la forme d'une dispersion/émulsion huile-dans-eau, contenant
a) au moins un composant lipidique ou cireux ayant un point de fusion > 50°C, n'ayant pas à être proportionnel aux composants b) ou c),
b) au moins un émulsifiant non ionique huile-dans-eau ayant une valeur HLB supérieure à 7 dans un mélange émulsifiant huile-dans-eau ayant une valeur HLB moyenne située dans la plage de 10 à 19,
c) au moins un émulsifiant non ionique eau-dans-huile ayant une valeur HLB de plus de 1,0 et inférieure ou égale à 7,0, qui peut former des structures cristallines liquides avec de l'eau seule ou avec de l'eau en présence d'un émulsifiant hydrophile, comme régulateur de consistance et/ou liant aqueux,
d) au moins une huile qui se présente sous une forme liquide à 20°C et qui n'est pas un composant parfumé ni une huile essentielle, dans laquelle l'écart maximum entre le paramètre de solubilité (moyen) de toutes les huiles constituantes d) et le paramètre de solubilité (moyen) de l'émulsifiant ou des émulsifiants eau-dans-huile est de -0,7 (cal/cm³)^{0,5} ou +0,7 (cal/cm³)^{0,5}, respectivement, en présence d'alcools gras saturés linéaires avec une longueur de chaîne d'au moins 8 atomes de carbone et -0,4 (cal/cm³)^{0,5} ou +0,7 (cal/cm³)^{0,5}, respectivement, en la présence d'émulsifiants eau-dans-huile autres que des alcools gras linéaires saturés avec une longueur de chaîne d'au moins 8 atomes de carbone, des alcools gras linéaires saturés avec une longueur de chaîne d'au moins 8 atomes de carbone étant absents et
la part des huiles de silicone et/ou hydrocarbures n'étant pas supérieure à 20 % par rapport au poids total des huiles, qui sont liquides à 20°C,
e) au moins un alcanol polyhydrique soluble dans l'eau en C₂-C₉ avec 2 à 6 groupes hydroxyle et/ou un polyéthylène glycol soluble dans l'eau ayant 3 à 20 motifs oxyde d'éthylène,
f) 5% jusqu'à moins de 50 % en poids d'eau, par rapport à la composition totale,
g) 1 % à 15 % en poids d'éthanol et/ou d'isopropanol, par rapport à la composition totale,
h) au moins une substance active déodorante ou anti-transpiration.

2. Bâtonnet déodorant ou anti-transpiration selon la revendication 1, le composant lipidique ou cireux a) étant choisi parmi les esters d'un alcanol en C₁₆-C₆₀ monohydrique saturé et d'un acide monocarboxylique en C₈-C₃₆ saturé, en particulier du béhénate de cétyle, du béhénate de stéaryle et du stéarate d'alkyle en C₂₀-C₄₀, des triesters de glycérine d'acides carboxyliques en C₁₂-C₃₀ linéaires saturés, qui peuvent être hydroxylés, de la cire de candelilla, de la cire de carnauba, de la cire d'abeille, des acides carboxyliques en C₁₄-C₃₆ linéaires saturés, ainsi que des mélanges des substances nommées ci-dessus.

3. Bâtonnet déodorant ou anti-transpiration selon l'une des revendications précédentes, dans lequel le composant lipidique ou cireux a) est contenu en tout en des quantités de 4 % à 20 % en poids, de préférence 8 % à 15 % en poids, par rapport à la composition totale.

4. Bâtonnet déodorant ou anti-transpiration selon l'une des revendications précédentes, dans lequel l'(les) ester(s) d'un alcanol en C₁₆-C₆₀ monohydrique saturé et d'un acide monocarboxylique en C₈-C₃₆ saturé est/sont contenu(s) en des quantités de 2 % à 10 % en poids, de préférence 2 % à 6 % en poids, par rapport à la composition totale.

5. Bâtonnet déodorant ou anti-transpiration selon l'une des revendications précédentes, l'émulsifiant non ionique huile-dans-eau b) ayant une valeur HLB supérieure à 7 étant choisi parmi
- les alcanols éthoxylés en C₈-C₂₄ avec une moyenne de 10 à 100 moles d'oxyde d'éthylène par mole,
- les acides carboxyliques éthoxylés en C₈-C₂₄ avec une moyenne de 10 à 100 moles d'oxyde d'éthylène par mole,
- les copolyols de silicone avec des motifs oxyde d'éthylène ou des motifs oxyde d'éthylène et oxyde de propylène,
- les mono- et oligoglycosides d'alkyle avec 8 à 22 atomes de carbone dans le résidu alkyle et leurs analogues éthoxylés,
- les stérols éthoxylés,
- les esters partiels de polyglycérines avec 2 à 10 motifs glycérine et estérifiés avec 1 à 4 résidu(s) d'acide gras en C₈-C₂₂ saturés ou insaturés, linéaires ou ramifiés, tant qu'ils présentent une valeur HLB supérieure à 7,
- ainsi que les mélanges des substances nommées ci-dessus.

6. Bâtonnet déodorant ou anti-transpiration selon l'une des revendications précédentes, l'émulsifiant huile-dans-eau b) étant contenu en une quantité totale de 0,5 % à 10 % en poids, de préférence 1 % à 4 % en poids, par rapport à la composition totale.

7. Bâtonnet déodorant ou anti-transpiration selon l'une des revendications précédentes, au moins un émulsifiant eau-dans-huile non ionique supplémentaire étant contenu, qui est choisi parmi
- les mono- et di-esters d'éthylène glycol d'acides carboxyliques en C₁₂-C₃₀ linéaires, saturés et insaturés, qui peuvent être hydroxylés, les mono-, di-, tri- et tétraesters pentaérythritylique d'acides carboxyliques en C₁₂-C₃₀ linéaires, saturés et insaturés, qui peuvent être hydroxylés, les alcanols en C₁₂-C₃₀ linéaires, saturés,
- les mono- et di-esters de glycéryle d'acides carboxyliques en C₁₂-C₃₀ linéaires, saturés et insaturés, qui peuvent être hydroxylés,
- les mono- et di-esters de propylène glycol d'acides carboxyliques en C₁₂-C₃₀ linéaires, saturés et insaturés, qui peuvent être hydroxylés,
- les mono-, di- et tri-esters de sorbitan d'acides carboxyliques en C₁₂-C₃₀ linéaires, saturés et insaturés, qui peuvent être hydroxylés,
- les mono- et di-esters de méthyl glucose d'acides carboxyliques en C₁₂-C₃₀ linéaires, saturés et insaturés, qui peuvent être hydroxylés,
- les stérols,
- les alcanols et les acides carboxyliques avec respectivement 8 à 24 atomes de carbone, en particulier avec 16 à 22 atomes de carbone, dans le groupe alkyle ou acyle et 1 à 4 motif(s) oxyde d'éthylène par molécule, qui montrent une valeur HLB supérieure à 1,0 et inférieure ou égale à 7,0,
- les mono-éthers glycéryliques d'alcools saturés et/ou insaturés, ramifiés et/ou non ramifiés d'une longueur de chaîne de 8 à 30, en particulier 12 à 18 atomes de carbone,
- les esters partiels de polyglycérines avec n = 2 à 10 motifs glycérine et estérifiées avec 1 à 5 résidu(s) d'acide gras en C₈-C₃₀ saturés ou insaturés, linéaires ou ramifiés, éventuellement hydroxylés, tant qu'ils présentent une valeur HLB inférieure ou égale à 7,
- ainsi que les mélanges des substances nommées ci-dessus.

8. Bâtonnet déodorant ou anti-transpiration selon l'une des revendications précédentes, l'émulsifiant eau-dans-huile c) étant choisi parmi le monostéarate d'éthylène glycol, le distéarate d'éthylène glycol, le monostéarate de pentaérythrityle, le distéarate de pentaérythrityle, le tristéarate de pentaérythrityle et le tétrastéarate de pentaérythrityle et leurs mélanges.

9. Bâtonnet déodorant ou anti-transpiration selon l'une des revendications précédentes, le(s) émulsifiant(s) eau-dans-huile c) étant contenu(s) en une quantité totale de 0,1 % à 15% en poids, de préférence 0,5% à 8,0 % en poids, et particulièrement préférée 1 % à 4 % en poids, par rapport à la composition totale.

10. Bâtonnet déodorant ou anti-transpiration selon l'une des revendications précédentes, l'huile d), liquide à 20°C, étant choisie parmi
- les esters d'acides di-carboxyliques d'alcanols en C₂-C₁₀ linéaires ou ramifiés,
- les alcools gras ramifiés, saturés ou insaturés avec 6 à 30 atomes de carbone,
- les triglycérides d'acides gras en C₈-C₃₀ linéaires ou ramifiés, saturés ou insaturés, éventuellement hydroxylés,
- les esters d'alcools gras ramifiés saturés ou insaturés avec 2 à 30 atomes de carbone avec des acides gras ramifiés, saturés ou insaturés avec 2 à 30 atomes de carbone, qui peuvent être hydroxylés,
- les produits d'addition de 1 à 5 motif(s) oxyde de propylène à des alcanols en C₈-C₂₂ monohydriques ou polyhydriques,
- les produits d'addition d'au moins 6 motifs oxyde de propylène à des alcanols en C₃-C₂₂ monohydriques ou polyhydriques,
- les esters d'alcool gras en C₈-C₂₂ d'acides hydroxycarboxyliques en C₂-C₇ monovalents ou polyvalents,
- les esters symétriques, asymétriques ou cycliques d'acide carbonique avec des alcools gras,
- les esters de dimères d'acides gras en C₁₂-C₂₂ insaturés (acides gras dimères) avec des alcanols en C₂-C₁₈ monohydriques linéaires, ramifiés ou cycliques ou avec des alcanols en C₂-C₆ linéaires ou ramifiés polyhydriques,
- ainsi que les mélanges des substances nommées ci-dessus.

11. Bâtonnet déodorant ou anti-transpiration selon l'une des revendications précédentes, l'(les) huile(s) d), liquide(s) à 20°C, étant contenue(s) en une quantité totale de 3 % à 20 % en poids, de préférence 5 % à 14 % en poids, par rapport au poids total de la composition.

12. Bâtonnet déodorant ou anti-transpiration selon l'une des revendications précédentes, l'(les) huile(s) d), liquide(s) à 20°C, contenant un maximum de 20 % en poids de l'(des) huile(s) par rapport au poids total des huiles liquides à 20°C, dont le paramètre de solubilité diffère de plus de -0,4 resp. -0,7 (cal/cm³)^{0,5} ou de plus de +0,7 (cal/cm³)^{0,5} du paramètre de solubilité (moyen) de l'(des) émulsifiant(s) eau-dans-huile c).

13. Bâtonnet déodorant ou anti-transpiration selon l'une des revendications précédentes, aucune des huiles liquides à 20°C n'étant contenue, dont le paramètre de solubilité diffère de plus de ±1,0 (cal/cm³)^{0,5} du paramètre de solubilité (moyen) de l'(des) émulsifiant(s) eau-dans-huile c).

14. Bâtonnet déodorant ou anti-transpiration selon l'une des revendications précédentes, l'alcanol en C₂-C₉ polyhydrique soluble dans l'eau e) avec 2 à 6 groupes hydroxyle étant choisi parmi le 1,2-propylène glycol, le 2-méthyl-1,3-propanediol, la glycérine, les butylène glycols tels que le 1,2-butylène glycol, le 1,3-butylène glycol et le 1,4-butylène glycol, les pentylène glycols tels que le 1,2-pentanediol et le 1,5-pentanediol, les hexanediols tels que le 1,6-hexanediol, les hexanetriols tels que le 1,2,6-hexanetriol, le 1,2-octanediol, le 1,8-octanediol, le dipropylène glycol, le tripropylène glycol, la diglycérine, la triglycérine, l'érythritol, le sorbitol ainsi que les mélanges des substances nommées ci-dessus.

15. Bâtonnet déodorant ou anti-transpiration selon l'une des revendications précédentes, l'alcanol en C₂-C₉ polyhydrique soluble dans l'eau avec 2 à 6 groupes hydroxyle et/ou le polyéthylène glycol soluble dans l'eau avec 3 à 20 motifs oxyde d'éthylène étant contenu en tout en des quantités de 3 % à 25 % en poids, de préférence 8 % à 18 % en poids.

16. Bâtonnet déodorant ou anti-transpiration selon l'une des revendications précédentes, au moins un composant lipidique ou cireux avec un point de fusion situé dans la plage de 25° à < 50°C étant en outre contenu, choisi parmi les mono-, di- et tri-esters de glycérine d'acide gras de coco, de *Butyrospermum parkii* (beurre de karité) et les esters d'alcools en C₈-C₁₈ monohydriques saturés avec des acides monocarboxyliques en C₁₂-C₁₈ saturés, ainsi que les mélanges de ces substances.

17. Bâtonnet déodorant ou anti-transpiration selon la revendication 16, le composant lipidique ou cireux avec un point de fusion situé dans la plage de 25° à < 50°C étant contenu en des quantités de 0,01 % à 20 % en poids, de préférence 3 % à 20 % en poids, de manière particulièrement préférée 5 % à 18 % en poids, et de manière extraordinairement préférée 6 % à 15 % en poids, par rapport à la composition totale.

18. Bâtonnet déodorant ou anti-transpiration selon l'une des revendications précédentes, la teneur totale en éthanol et/ou isopropanol étant de 2 % à 10 % en poids, de manière particulièrement préférée 3 % à 8 % en poids, de manière exceptionnellement préférée 5 % à 7 % en poids, respectivement par rapport à la composition totale.

19. Bâtonnet déodorant ou anti-transpiration selon l'une des revendications précédentes, la substance active déodorante étant choisie parmi
- les inhibiteurs de l'arylsulfatase, les inhibiteurs de la β-glucuronidase, les inhibiteurs de l'aminoacylase, les inhibiteurs de l'estérase, les inhibiteurs de la lipase et les inhibiteurs de la lipoxigénase,
- les éthers d'α-monoalkyl glycérine avec un résidu alkyle en C₆-C₂₂ ramifié ou linéaire, saturé ou insaturé, éventuellement hydroxylé, en particulier l'éther d'α-(2-éthylhexyl)glycérine,
- le phénoxyéthanol,
- les huiles parfumées inhibitrices de germes,
- les huiles parfumées Deosafe^{®},
- les composants ayant une action pré-biotique,
- les esters d'acide trialkylcitrique, en particulier le citrate de triéthyle,
- les substances qui réduisent le nombre de bactéries de la peau générant des odeurs du groupe des staphylocoques, de *corynebacterium xerosis*, de *corynebacterium,* des bactéries entérocoques et microcoques, resp. qui inhibent leur développement,
- des composés de type zinc, en particulier du sulfonate phénolique de zinc et du ricinoléate de zinc,
- les composés organo-halogènes, en particulier le triclosan, la chlorhexidine, le gluconate de chlorhexidine et les halogénures de benzalconium,
- les composés d'ammonium quaternaire, en particulier le chlorure de cétylpyridinium,
- les agents absorbant les odeurs, en particulier les silicates et les zéolites,
- le bicarbonate de sodium,
- les lanthibiotiques,
- ainsi que les mélanges des substances nommées ci-dessus,
- et/ou la substance active anti-transpiration étant choisie parmi les sels solubles dans l'eau, astringents, inorganiques et organiques d'aluminium, de zirconium et de zinc, respectivement n'importe quels mélanges de ces sels.

20. Bâtonnet déodorant ou anti-transpiration selon l'une des revendications précédentes, la substance active déodorante, étant contenue en une quantité totale de 0,1 % à 10 % en poids, de préférence 0,2 % à 7% en poids, particulièrement préférée 0,3 % à 5 % en poids et exceptionnellement préférée 0,4% à 1,0 % en poids, par rapport à 0,1 % à 10 % en poids, et/ou la substance active anti-transpiration étant contenue en une quantité totale de 3 % à 25 % en poids, de préférence 5 % à 22 % en poids, et en particulier 10 % à 20 % en poids, par rapport respectivement au poids total de la substance active dans la composition totale.

21. Bâtonnet déodorant ou anti-transpiration selon l'une des revendications précédentes, au moins une charge sous forme de particule, insoluble dans l'eau, solide étant en outre contenue.

22. Bâtonnet déodorant ou anti-transpiration selon l'une des revendications précédentes, la charge sous forme de particule, insoluble dans l'eau, solide, étant choisie parmi les amidons et les dérivés d'amidon - éventuellement modifiés -, qui sont, si cela est souhaité, pré-gélatinisés, la cellulose et les dérivés de cellulose, le dioxyde de silicium, les acides siliciques, les particules sphériques de polyalkylsesquisiloxane, les gels siliciques (de silice), le talc, le kaolin, les argiles, par exemple les bentonites, les silicates de magnésium aluminium, le nitrure de bore, les dérivés de lactoglobuline, la poudre de verre, les poudres polymères ainsi que les mélanges des substances nommées ci-dessus.

23. Bâtonnet déodorant ou anti-transpiration selon l'une des revendications précédentes, la charge sous forme de particule, insoluble dans l'eau, solide, étant contenue en une quantité totale de 0,01 % à 30 % en poids, de préférence 5 % à 20 % en poids, de manière particulièrement préférée 8 % à 15 % en poids, par rapport respectivement à la composition totale.

24. Bâtonnet déodorant ou anti-transpiration selon l'une des revendications précédentes, 10 % à moins de 30 % en poids d'eau, par rapport à la composition totale étant contenue.

25. Bâtonnet déodorant ou anti-transpiration selon l'une des revendications précédentes, la teneur totale en émulsifiants et/ou tensioactifs non ioniques et ioniques ayant une valeur HLB supérieure à 8 avec un maximum de 20 % en poids, de préférence un maximum de 15% en poids, de manière particulièrement préférée un maximum de 10 % en poids, de manière particulièrement préférée un maximum de 7% en poids, de manière particulièrement préférée en outre un maximum de 4 % en poids et de manière exceptionnellement préférée un maximum de 3 % en poids, par rapport respectivement à la composition totale de l'invention.

26. Composition pour bâtonnet selon l'une des revendications précédentes, **caractérisée par** une valeur de force de pénétration située dans la plage de 150 à 800 grammes force (g-force), 250 à 600 grammes force (g-force), de manière particulièrement préférée 300 à 520 grammes force (g-force), de manière extraordinairement préférée 340 à 500 grammes force (g-force), à une profondeur de pénétration de 5,000 mm.

27. Composition pour bâtonnet selon l'une des revendications précédentes, **caractérisée par** une résistance électrique d'un maximum de 300 kΩ, de préférence un maximum de 100 kΩ et de manière particulièrement préférée un maximum de 80 kΩ.

28. Bâtonnet déodorant ou anti-transpiration selon l'une des revendications précédentes, au moins un composant parfumé étant en outre contenu.

29. Bâtonnet déodorant ou anti-transpiration selon l'une des revendications précédentes, au moins une substance de capture de radicaux étant contenue.

30. Bâtonnet déodorant ou anti-transpiration selon l'une des revendications précédentes, au moins un filtre UV étant contenu.

31. Bâtonnet déodorant ou anti-transpiration selon l'une des revendications précédentes, au moins une substance complexante étant contenue.

32. Bâtonnet déodorant ou anti-transpiration selon l'une des revendications précédentes, au moins une substance inhibitrice de la croissance des poils étant contenue.

33. Procédé non thérapeutique, cosmétique, de réduction de l'odeur corporelle, une composition cosmétique déodorante et/ou anti-transpiration selon l'une des revendications 1 à 32 étant appliquée sur la peau, en particulier sur la peau des aisselles.

34. Procédé de fabrication d'un bâtonnet déodorant ou anti-transpiration selon l'une des revendications 1 à 32, moyennant quoi les composants cireux et huileux conjointement à l'(aux) émulsifiant(s) huile-dans-eau et eau-dans-huile étant chauffé(s) à 90°à 95°C et fondu(s), suite à quoi l'eau également chauffée à 90° à 95°C est ajoutée conjointement aux substances et ingrédients actifs solubles dans l'eau, tout en étant vigoureusement agités ; les ingrédients supplémentaires étant éventuellement mélangés dedans, le mélange étant refroidi à une température de remplissage appropriée, chargé dans des distributeurs appropriés et solidifié à la température ambiante par refroidissement statique (sans agitation supplémentaire).
